# EUROPEAN PATENT SPECIFICATION

(11) **EP 3 252 153 B1**
(45) Date of publication and mention of the grant of the patent: **16.09.2026**
(21) Application number: 16743340.8
(22) Date of filing: 26.01.2016
(51) Int. Cl.: C12N 5/10, C12N 5/0735, C12M 3/02

(54) **METHOD FOR PRODUCING CELL AGGREGATES**
VERFAHREN ZUR HERSTELLUNG VON ZELLAGGREGATEN
PROCÉDÉ DE PRODUCTION D'AGRÉGATS CELLULAIRES

(30) Priority: 29.01.2015 JP 2015015306
(43) Date of publication of application: 06.12.2017
(73) Proprietor: KANEKA CORPORATION, Osaka-shi, Osaka 530-8288 (JP); THE UNIVERSITY OF TOKYO, Tokyo 113-8654 (JP)
(72) Inventor: HORIGUCHI, Ikki, Tokyo 113-8654 (JP); SAKAI, Yasuyuki, Tokyo 113-8654 (JP); IBUKI, Masato, Takasago-shi Hyogo 676-8688 (JP)
(74) Representative: Hoffmann Eitle
(86) International application number: PCT/JP2016/052128
(87) International publication number: WO 2016/121737

(56) References cited:
- WO-A1-2014/133170
- JP-A- 2006 505 248
- JP-A- 2008 099 662
- US-A1- 2004 241 839
- HARADA JUN ET AL: "Sphingosine-1-phosphate induces proliferation and morphological changes of neural progenitor cells", JOURNAL OF NEUROCHEMISTRY, WILEY INTERSCIENCE, NEW YORK, NY, US, vol. 88, no. 4, 1 February 2004 (2004-02-01), pages 1026 - 1039, XP002605006, ISSN: 0022-3042, DOI: 10.1046/J.1471-4159.2003.02219.X
- YANAGIDA K ET AL: "LPA4/p2y9/GPR23 mediates rho-dependent morphological changes in a rat neuronal cell line", JOURNAL OF BIOLOGICAL CHEMISTRY 20070223 AMERICAN SOCIETY FOR BIOCHEMISTRY AND MOLECULAR BIOLOGY INC. US, vol. 282, no. 8, 23 February 2007 (2007-02-23), pages 5814 - 5824, XP002781626, DOI: 10.1074/JBC.M610767200
- HONG G ET AL: "Sphingosine-1-phosphate modulates growth and adhesion of ovarian cancer cells", FEBS LETT, ELSEVIER, AMSTERDAM, NL, vol. 460, no. 3, 5 November 1999 (1999-11-05), pages 513 - 518, XP004260500, ISSN: 0014-5793, DOI: 10.1016/S0014-5793(99)01400-3
- HORIGUCHI IKKI ET AL: "Serum replacement with albumin-associated lipids prevents excess aggregation and enhances growth of induced pluripotent stem cells in suspension culture.", BIOTECHNOLOGY PROGRESS 08 07 2016, vol. 32, no. 4, 8 July 2016 (2016-07-08), pages 1009 - 1016, XP002781627, ISSN: 1520-6033
- FRISCA F. ET AL.: "Rho/ROCK pathway is essential to the expansion, differentiation, and morphological rearrangements of human neural stem/progenitor cells induced by lysophosphatidic acid", J LIPID RES, vol. 54, no. 5, May 2013 (2013-05-01), pages 1192 - 1206, XP055472085
- BREWER GJ. ET AL.: "Optimized survival of hippocampal neurons in B27-supplemented Neurobasal, a new serum-free medium combination.", J NEUROSCI RES., vol. 35, no. 5, pages 567 - 76, XP001097842
- GARCIA-GONZALO FR . ET AL.: "Albumin-associated lipids regulate human embryonic stem cell self- renewal.", PLOS ONE., vol. 3, no. 1, December 2008 (2008-12-01), pages e1384 ., XP002548861
- AVERY K. ET AL.: "Sphingosine-1-phosphate mediates transcriptional regulation of key targets associated with survival, proliferation, and pluripotency in human embryonic stem cells.", STEM CELLS DEV., 17 December 2008 (2008-12-17), pages 1195 - 206, XP055472091
- RYU JM. ET AL.: "Sphingosine-1-phosphate-induced Flk-1 transactivation stimulates mouse embryonic stem cell proliferation through S1P1/S1P3-dependent beta-arrestin/c-Src pathways.", STEM CELL RES., vol. 12, no. 1, 7 September 2013 (2013-09-07), pages 69 - 85, XP028671035

## Description

### Technical Field

The present invention relates to a method for producing cell aggregates such that useful cells such as pluripotent stem cells are cultured in suspension in a liquid culture medium.

### Background Art

Recent research on human pluripotent stem cells (e.g., human ES cells and human iPS cells) has increasingly made regenerative medicine come in reality. These cells possess an ability to proliferate infinitely and an ability to differentiate into various types of cells. Thus, regenerative medicine using the pluripotent stem cells should radically change therapeutic interventions against, for example, refractory diseases and lifestyle-related diseases. It has already been possible that the pluripotent stem cells can be induced and differentiated *in vitro* into various types of cells including neurons, cardiomyocytes, blood cells, and retinal cells.

One of objectives directed toward practical use of regenerative medicine in which pluripotent stem cells are used to regenerate a variety of organs involves how a large number of cells necessary for regeneration of organs can be produced efficiently. For example, the regeneration of a liver requires about 2 × 10¹¹ cells. A substrate plate with an area of 10⁶ cm² or more is needed so as to culture the above number of cells using adherent culture on a flat substrate plate. This means that about 20,000 common 10-cm dishes are needed. Because the number of cells to be obtained using adherent culture on a surface of the substrate plate depends on the surface area of the culture plate, it is difficult to scale up the culture. Accordingly, it is hard to provide an enough number of cells to make regenerative medicine available.

It is easy to scale up suspension culture in which cells are cultured in suspension in a liquid culture medium. Hence, the suspension culture should be fit for mass production of cells.

Non-Patent Literature 3 discloses a process for producing spheroids with a uniform size, the process comprising: using a spinner flask as cell cultureware for suspension culture; and culturing human pluripotent stem cells in suspension while strongly stirring a liquid culture medium.

Non-Patent Literature 4 discloses a process for producing spheroids with a uniform size in each micro-well, the process comprising using a substrate plate on which small micro-wells are formed.

Non-Patent Literature 5 discloses a culturing method comprising: using a culture medium the viscosity and specific gravity of which is adjusted; keeping pluripotent stem cells in suspension; and reducing a collision between the cells.

Patent Literature 1 discloses a technology in which cells are cultured while being subjected to rotary shaking culture in a liquid culture medium, so that cell aggregates are produced.

Patent Literature 2 discloses a method in which pluripotent stem cells are cultured in suspension until the average diameter of cell aggregates reaches about 200 to 300 µm.

### Citation List

### Patent Literature

Patent Literature 1: JP Patent Publication (Kokai) No. 2003-304866A
Patent Literature 2: WO2013/077423A

### Non-Patent Literature

Non-Patent Literature 1: Takayama N, Nishimura S, Nakamura S, Shimizu T, Ohnishi R, Endo H, Yamaguchi T, Otsu M, Nishimura K, Nakanishi M, Sawaguchi A, Nagai R, Takahashi K, Yamanaka S, Nakauchi H, Eto K. Transient activation of c-MYC expression is critical for efficient platelet generation from human induced pluripotent stem cells. J Exp Med. 2010 Dec 20; 207(13): 2817-30. doi: 10.1084/jem.20100844. Epub 2010 Nov 22. PubMed PMID: 21098095; PubMed Central PMCID: PMC3005234.
Non-Patent Literature 2: FR Garcia-Gonzalo and JCI Belmonte, Albumin-Associated Lipids Regulate Human Embryonic Stem Cell Self-Renewal. PLoS ONE, 2008, 3(1), e1384
Non-Patent Literature 3: Olmer R. et al., Tissue Engineering: Part C, Volume 18 (10): 772-784 (2012)
Non-Patent Literature 4: Ungrin MD et al., PLoS ONE, 2008, 3(2), e1565
Non-Patent Literature 5: Otsuji GT et al., Stem Cell Reports, Volume 2: 734-745 (2014)

### Summary of Invention

### Technical Problem

As described above, various kinds of technology for preparing cell aggregates using suspension culture have been developed. However, the present inventors have thought of there being the following problems that still need to be solved.

Adherent cells (e.g., pluripotent stem cells) can form aggregates while cultured in suspension. Examples of the mechanism of cell aggregation include: non-specific cell-to-cell attachment mediated by membrane proteins and/or plasma membranes; and intercellular adhesion mediated by cadherins on the cell surface. Because cells such as human iPS cells cannot survive as single cells, it is necessary for the cells to form cell aggregates for survival. However, when the size of the aggregates is too large, nutrients cannot be sufficiently distributed into cells located deep inside the aggregates, which causes problems including inhibition of their proliferation and difficulty to maintain an undifferentiated state. To prevent excessive aggregation, it seems effective to make a liquid culture medium flow during culture. The excessive flow, however, could damage cells due to physical stimulation to cells. Here, a suspension culture technology has been sought that is used to produce aggregates with an appropriate size without damaging cells. The conventional methods for producing aggregates listed in the Background Art section still have room for improvement.

The process of Non-Patent Literature 3 likely causes cells to die due to shear stress, which is a defect of the process.

In the process of Non-Patent Literature 4, it is difficult to scale up a culture and to change a culture medium.

In the method of Non-Patent Literature 5, because of less movement of a culture medium during culture, nutritional components are less likely to be supplied to cell aggregates.

Patent Literature 1 fails to disclose a means for controlling the size of cell aggregates to an appropriate size.

Patent Literature 2 discloses adding, to a culture medium, an aqueous polymer as a means for preventing adhesion between cell aggregates, so that the viscosity increases. This causes the same defect as in the case of Non-Patent Literature 5, in which oxygen and nutritional components are less likely to be supplied to cell aggregates.

Here, the purpose of the present invention is to provide a method for producing cell aggregates using suspension culture, wherein it is easy to control the size of the cell aggregates so as to be appropriate for culturing, and the likelihood of damaging the cells is low.

### Solution to Problem

The present inventors have obtained a surprising finding where when a lipid (in particular, a phospholipid) is added to a liquid culture medium and cells are cultured in suspension in the medium, a large amount of population of cell aggregates with an appropriate size can be produced. Based on this finding, the present inventors have completed the present invention. The present invention does not rely on mechanical/physical means, i.e., modifying culturing conditions, such as the viscosity of a culture medium, shear stress due to stirring, or the shape of cultureware such as a microwell plate. The present invention uses biochemical/chemical means, i.e., modifying the composition of a culture medium by using a substance present *in vivo.* Specifically, the present invention encompasses the following aspects.

### [Item 1]

A method for producing cell aggregates, comprising a step of culturing cells while suspended in a liquid culture medium comprising a lysophospholipid,
wherein the cells are pluripotent stem cells, wherein the liquid culture medium comprises the lysophospholipid in an amount of more than 0.0064 µg/mL and 100 µg/mL or less,
wherein the lysophospholipid is at least one of lysophosphatidic acid and sphingosine-1-phosphoric acid.

### [Item 2]

The method according to item 1, further comprising at least one of a step of adding the lysophospholipid to prepare the liquid culture medium and a step of generating the lysophospholipid through an enzymatic reaction to prepare the liquid culture medium.

### [Item 3]

The method according to any one of items 1 or 2, wherein the cells are cells isolated after undergoing adherent or suspension culture.

### [Item 4]

The method according to any one of items 1 to 3, wherein the liquid culture medium comprises at least one selected from the group consisting of L-ascorbic acid, insulin, transferrin, selenium, and sodium bicarbonate.

### [Item 5]

The method according to any one of items 1 to 4, wherein the liquid culture medium comprises a growth factor.

### [Item 6]

The method according to item 5, wherein the growth factor is at least one of FGF2 and TGF-β1.

### [Item 7]

The method according to any one of items 1 to 6, wherein the liquid culture medium comprises a ROCK inhibitor.

### [Item 8]

Use of a lysophospholipid for inhibition of cell aggregation,
wherein the cells are pluripotent stem cells, wherein the lysophospholipid is present in a concentration of more than 0.0064 µg/mL and 100 µg/mL or less,
wherein the lysophospholipid is at least one of lysophosphatidic acid and sphingosine-1-phosphoric acid.

### [Item 9]

The use according to item 8, wherein the lysophospholipid is used in combination with a growth factor.

### [Item 10]

The use according to items 9, wherein the growth factor is at least one of FGF2 and TGF-β1.

### [Item 11]

The use according to any one of items 8 to 10, wherein the lysophospholipid is used in combination with a ROCK inhibitor.

### Advantageous Effects of Invention

The method of the present invention allows for mass production of cell aggregates that are fit for suspension culture and makes it possible to produce a large number of relevant cells.

### Brief Description of the Drawings

[Figure 1] Figure 1 schematically illustrates an outline of a protocol for producing cell aggregates.
[Figure 2] Figure 2 shows three-dimensional aggregates that were produced from human iPS cells by rotary shaking culture with different KSR concentrations in a container non-adherent to cells (at Day 2 of culture). % = volume (v) / volume (v).
[Figure 3] Figure 3 shows the time course of change in glucose consumption when KSR at each concentration was added. n = 4.
[Figure 4] Figure 4 shows the number of cells at Day 5 of culture when KSR at each concentration was added. n = 4.
[Figure 5] Figure 5 specifies components of KSR disclosed in Non-Patent Literature 2.
[Figure 6] Figure 6 shows how addition of different factors (AlbuMAX^{™} II, BSA, insulin, and transferrin) affected the formation of aggregates. % = weight (w) / volume (v).
[Figure 7] Figure 7 indicates the percentage of human iPS cells expressing OCT 4 when subjected to adherent culture or suspension culture (containing aggregates).
[Figure 8] Figure 8 is micrographs obtained when a suspension containing human iPS cells was used for suspension culture while lipid-free bovine serum albumin and different lipids were added.
[Figure 9] Figure 9 is micrographs obtained when a suspension containing human iPS cells was used for suspension culture while lipid-free bovine serum albumin and LPA (lysophosphatidic acid) at each concentration were added. φ represents the average (± standard deviation) of the size of cell aggregates.
[Figure 10] Figure 10 is micrographs obtained when a suspension containing human iPS cells was used for suspension culture while lipid-free bovine serum albumin and S1P (sphingosine-1-phosphoric acid) at each concentration were added. φ represents the average (± standard deviation) of the size of cell aggregates.
[Figure 11] Figure 11 is micrographs obtained when a suspension containing human iPS cells was used for suspension culture while lipid-free bovine serum albumin alone, or additional LPA or S1P at each concentration was added.
[Figure 12] Figure 12 shows the time course of change in glucose consumption when a suspension containing human iPS cells was used for suspension culture while lipid-free bovine serum albumin alone, or additional LPA or S1P at each concentration was added. n = 3.
[Figure 13] Figure 13 shows a total cell count at Day 5 of culture when a suspension containing human iPS cells was used for suspension culture while lipid-free bovine serum albumin alone, or additional LPA or S1P at each concentration was added. n = 3.
[Figure 14] Figure 14 shows the percentage of human iPS cells positive for undifferentiation markers (OCT4 and SOX2) when the cells were subjected to adherent culture or suspension culture using a cell suspension containing 1.0 µg/mL of LPA or S1P.
[Figure 15] Figure 15 is micrographs obtained when the cells were cultured in suspension at each culture scale (4-mL scale, 300-mL scale, and 1.6-L scale) 1 day after seeding (Day 1) and 5 or 6 days after seeding (Day 5 or Day 6).
[Figure 16] Figure 16 shows the time course of change in cell density when the cells were cultured in suspension at each culture scale (4-mL scale, 300-mL scale, and 1.6-L scale).
[Figure 17] Figure 17 shows the percentage of cells positive for undifferentiation markers (OCT4 and SOX2) when the cells were subjected to adherent culture or suspension culture at each culture scale (4-mL scale, 300-mL scale, and 1.6-L scale).

### Description of Embodiments

Hereinafter, preferable embodiments of the present invention will be described in detail.

### <1. Cells>

Aggregate-forming cells, which are cultured by the method of an embodiment of the present invention are pluripotent stem cells as recited in the appended claims. Examples of the cells may include: animal-derived cells; preferably mammalian-derived cells; more preferably biological tissue-derived cells and cells derived from the biological tissue-derived cells; particularly preferably epithelial tissue-derived cells and cells derived from the epithelial tissue-derived cells, connective tissue-derived cells and cells derived from the connective tissue-derived cells, muscular tissue-derived cells and cells derived from the muscular tissue-derived cells, or nervous tissue-derived cells and cells derived from the nervous tissue-derived cells; further more preferably animal-derived stem cells and cells differentiated from the animal-derived stem cells; still more preferably animal-derived pluripotent stem cells and cells differentiated from the animal-derived pluripotent stem cells; still more preferably mammalian-derived pluripotent stem cells and cells differentiated from the mammalian-derived pluripotent stem cells; and most preferably human-derived pluripotent stem cells and cells differentiated from the human-derived pluripotent stem cells.

As used herein, the term "pluripotent stem cells" refers to cells that are pluripotent (multipotent) cells which can differentiate into all types of cells constituting a living body and that can continue proliferating infinitely while maintaining their pluripotent state during *in vitro* culture under suitable conditions. Specific examples of the pluripotent stem cells include, but are not limited to, embryonic stem cells (ES cells), EG cells, which are pluripotent stem cells derived from fetal primordial germ cells, (Shamblott M. J. et al., Proc. Natl. Acad. Sci. USA. (1998) 95, p.13726-13731), GS cells, which are testis-derived pluripotent stem cells, (Conrad S., Nature (2008) 456, p.344-349), and iPS cells (induced pluripotent stem cells), which are somatic cell-derived induced pluripotent stem cells. Regarding the pluripotent stem cells used in the present invention, particularly preferred are ES cells or iPS cells. iPS cells are cultured cells produced by introducing reprogramming factors into a somatic cell, so that the somatic cell is reprogrammed into an undifferentiated state and is given pluripotency. Examples of the reprogramming factors that can be used include OCT3/4, KLF4, SOX2, and c-Myc (Yu J, et al. Science. 2007; 318:1917-20). For example, OCT3/4, SOX2, LIN28, and Nanog may be used (Takahashi K, et al. Cell. 2007; 131:861-72). Examples of how to introduce these factors into a cell include, but are not particularly limited to, a plasmid-mediated gene transfer, synthetic RNA introduction, and a direct injection of a protein(s). In addition, it may be possible to use iPS cells that are created using, for example, microRNA, RNA, and/or a low-molecular-weight compound. As the pluripotent stem cells concern iPS cells, etc.), commercially available products or cells obtained from a third party may be used or freshly prepared ones may be used. Examples of iPS cell lines that can be used include 253G1, 201B6, 201B7, 409B2, 454E2, HiPS-RIKEN-1A, HiPS-RIKEN-2A, HiPS-RIKEN-12A, Nips-B2, TkDN4-M, TkDA3-1, TkDA3-2, TkDA3-4, TkDA3-5, TkDA3-9, TkDA3-20, hiPSC 38-2, MSC-iPSC1, and BJ-iPSC1. Also, freshly prepared clinical-grade iPS cells may be used. Examples of the origin of cells when iPS cells are created include, but are not particularly limited to, fibroblasts and lymphocytes.

Cells used in the present invention may be originated from any animal. Examples of the origin may include: mammals such as rodents (e.g., a mouse, rat, hamster), primates (e.g., a human, gorilla, chimpanzee), and domestic animals and pets (e.g., a dog, cat, rabbit, cow, horse, sheep, goat). Particularly preferred are human cells.

In the present invention, cells isolated after undergoing adherent or suspension culture may be used. Here, the term "isolated cells" means cells obtained by detaching and dispersing a cell population composed of a plurality of cells adhering to one another. The isolation involves the step of detaching and dispersing cells adhering to, for example, cultureware and/or a culture support or a cell population, in which cells adhere to one another, to give single cells. The cell population to be isolated may be in suspension in a liquid culture medium. Preferable examples of the isolation procedure may include, but are not particularly limited to, a procedure using a detachment agent (e.g., a cell detachment enzyme such as trypsin or collagenase), a chelating agent (e.g., EDTA (ethylene diamine tetraacetic acid)), or a mixture of the detachment agent and the chelating agent. Examples of the detachment agent include, but are not particularly limited to, trypsin, Accutase (a registered trade mark), TrypLE^{™} Express Enzyme (Life Technologies Japan Ltd.), TrypLE^{™} Select Enzyme (Life Technologies Japan Ltd.), "Dispase" (a registered trade mark), and collagenase. The cells that have been isolated, frozen, and stored after the isolation procedure may be preferably used in the present invention.

### <2. Cell Aggregates>

As used herein, a cell aggregate refers to what is called a spheroid that is a clustered body formed while a plurality of cells aggregate three-dimensionally. Cell aggregates prepared in accordance with an embodiment of the present invention typically have a substantially spherical shape.

As used herein, aggregate-forming cells have no particular limitation as long as they contain at least one type of the above adherent cells. For example, cells expressing a pluripotent stem cell marker are included in the cell aggregate composed of pluripotent stem cells (e.g., human pluripotent stem cells, human embryonic stem cells). Examples of the pluripotent stem cell maker include alkaline phosphatase, NANOG, OCT4, SOX2, TRA-1-60, c-Myc, KLF4, LIN28, SSEA-4, and SSEA-1. When the aggregate-forming cells are the pluripotent stem cells, the percentage of cells expressing the pluripotent stem cell marker may be preferably 80% or higher, more preferably 90% or higher, more preferably 91% or higher, more preferably 92% or higher, more preferably 93% or higher, more preferably 94% or higher, more preferably 95% or higher, more preferably 96% or higher, more preferably 97% or higher, more preferably 98% or higher, more preferably 99% or higher, and more preferably 100% or less.

The size of cell aggregates as produced by the method of the present invention has no particular limitation. When observed under a microscope, the upper limit of the size of the widest portion on a micrograph is preferably 1000 µm, more preferably 900 µm, still more preferably 800 µm, still more preferably 700 µm, further more preferably 600 µm, still more preferably 500 µm, still more preferably 400 µm, and still more preferably 300 µm. The lower limit is preferably 50 µm, more preferably 60 µm, further more preferably 70 µm, still more preferably 80 µm, still more preferably 90 µm, and most preferably 100 µm. The cell aggregates with such a size range have a preferable cell growth environment because oxygen and nutritional components are easily supplied to their inner cells.

When measured by weight, 10% or higher, more preferably 20% or higher, still more preferably 30% or higher, still more preferably 40% or higher, still more preferably 50% or higher, still more preferably 60% or higher, still more preferably 70% or higher, still more preferably 80% or higher, and most preferably 90% or higher of the cell aggregates constituting a cell aggregate population prepared by the method of the present invention may have a size within the above range.

### <3. Liquid Culture Medium>

Regarding a liquid culture medium used in the present invention, any of liquid culture media for culturing an animal cell may be used as a basal medium. The liquid culture medium of interest may be prepared by appropriately adding, if necessary, another component.

Examples of the basal medium that can be used include, but are not particularly limited to, BME medium, BGJb medium, CMRL1066 medium, Glasgow MEM medium, Improved MEM Zinc Option medium, IMDM medium (Iscove's Modified Dulbecco's Medium), Medium 199 medium, Eagle MEM medium, αMEM medium, DMEM medium (Dulbecco's Modified Eagle's Medium), Ham's F10 medium, Ham's F12 medium, RPMI 1640 medium, Fischer's medium, and a mixed medium thereof (e.g., DMEM/F12 medium (Dulbecco's Modified Eagle's Medium/Nutrient Mixture F-12 Ham)). The DMEM/F12 medium may be used, in particular, by mixing DMEM medium and Ham's F12 medium in a weight ratio of preferably from 60/40 to 40/60, more preferably from 55/45 to 45/55, and most preferably 50/50.

The liquid culture medium used in the present invention is preferably a medium containing no serum, namely a serum-free medium.

The liquid culture medium used in the present invention preferably contains at least one selected from L-ascorbic acid, insulin, transferrin, selenium, and sodium bicarbonate, and more preferably contains all of them. The L-ascorbic acid, insulin, transferrin, selenium, and sodium bicarbonate may be added to the medium in the form of, for example, a solution, derivative, salt, or mixed reagent. For example, L-ascorbic acid may be added to the medium in the form of a derivative such as magnesium-ascorbyl-2-phosphate. Selenium may be added to the medium in the form of a selenite (e.g., sodium selenite). The insulin and transferrin may be natural ones isolated from a tissue or serum of an animal (e.g., preferably a human, mouse, rat, cow, horse, goat). They may be genetically engineered recombinant proteins. The insulin, transferrin, and selenium may be added to the medium in the form of a reagent ITS (insulin-transferrin-selenium). The ITS is a cell growth-promoting additive containing insulin, transferrin, and sodium selenite.

A commercially available culture medium containing at least one selected from L-ascorbic acid, insulin, transferrin, selenium, and sodium bicarbonate may be used as a liquid culture medium of the present invention. Examples of a commercially available culture medium supplemented with insulin and transferrin may include CHO-S-SFM II (Life Technologies Japan Ltd.), Hybridoma-SFM (Life Technologies Japan Ltd.), eRDF Dry Powdered Media (Life Technologies Japan Ltd.), UltraCULTURE^{™} (BioWhittaker, Inc.), UltraDOMA^{™} (BioWhittaker, Inc.), UltraCHO^{™} (BioWhittaker, Inc.), and UltraMDCK^{™} (BioWhittaker, Inc.). For, example, STEMPRO (a registered trademark), hESC SFM (Life Technologies Japan Ltd.), mTeSR1 (Veritas, Ltd.), or TeSR2 (Veritas, Ltd.) may be preferably used. In addition, it is preferable to use a liquid culture medium used for culturing human iPS cells and/or human ES cells.

The liquid culture medium used in the present invention preferably contains at least one growth factor. The liquid culture medium preferably contains at least one growth factor, which is not limited to the following, selected from the group consisting of FGF2 (basic fibroblast growth factor-2), TGF-β1 (transforming growth factor-β1), Activin A, IGF-1, MCP-1, IL-6, PAI, PEDF, IGFBP-2, LIF, and IGFBP-7. Particularly preferred growth factor is FGF2 and/or TGF-β1.

The most preferable liquid culture medium used in the present invention is a serum-free medium containing, in addition to albumin and/or the below-described lipid, components: L-ascorbic acid, insulin, transferrin, selenium, and sodium bicarbonate as well as at least one growth factor. Particularly preferred is serum-free DMEM/F12 medium containing L-ascorbic acid, insulin, transferrin, selenium, and sodium bicarbonate as well as at least one growth factor (preferably, FGF2 and TGF-β1). Examples of such a medium that can be preferably used include Essential 8^{™} medium (Life Technologies Japan Ltd.) supplemented with albumin and/or the below-described lipid. The Essential 8^{™} medium may be prepared by mixing DMEM/F-12 (HAM) (1:1), which is a DMEM/F12 medium marketed by Life Technologies Japan Ltd., and Essential 8^{™} supplement (containing L-ascorbic acid, insulin, transferrin, selenium, sodium bicarbonate, FGF2, and TGF-β1).

The liquid culture medium used in the present invention may contain components such as fatty acids or lipids, amino acids (e.g., non-essential amino acids), vitamins, cytokines, antioxidants, 2-mercaptoethanol, pyruvic acid, buffers, inorganic salts, antibiotics, and kinase inhibitors.

Examples of the antibiotics that can be used include penicillin, streptomycin, and amphotericin B.

Examples of the kinase inhibitors that can be added include ROCK inhibitors.

The ROCK inhibitors are defined as a substance that inhibits the kinase activity of Rho kinase (ROCK, a Rho-associated protein kinase). Examples include: Y-27632 (4-[(1R)-1-aminoethyl]-N-pyridine-4-ylcyclohexane-1-carboxamide) or a dihydrochloride thereof (see, for example, Ishizaki et al., Mol. Pharmacol. 57, 976-983 (2000); Narumiya et al., Methods Enzymol. 325,273-284 (2000)); Fasudil/HA1077 (1-(5-isoquinoline sulfonyl)homopiperazine) or a dihydrochloride thereof (see, for example, Uenata et al., Nature 389: 990-994 (1997)); H-1152((S)-(+)-2-methyl-1-[(4-methyl-5-isoquinolinyl)sulfonyl]-hexahydro-1H-1,4-diazepine) or a dihydrochloride thereof (see, for example, Sasaki et al., Pharmacol. Ther. 93: 225-232 (2002)); Wf-536((+)-(R)-4-(1-aminoethyl)-N-(4-pyridyl)benzamide monohydrochloride) (see, for example, Nakajima et al., Cancer Chemother. Pharmacol. 52(4): 319-324 (2003)) and a derivative thereof; and antisense nucleic acid against ROCK, RNA interference-inducing nucleic acid (e.g., siRNA), a dominant negative mutant, and vectors expressing these molecules. In addition, because other low-molecular-weight compounds have been known as the ROCK inhibitors, such compounds or derivatives thereof may be used in accordance with the present invention (see, for example, US Patent Application Publication Nos. 20050209261, 20050192304, 20040014755, 20040002508, 20040002507, 20030125344, and 20030087919, and WO2003/062227, WO2003/059913, WO2003/062225, WO2002/076976, and WO2004/039796). In the present invention, at least one kind of the ROCK inhibitor may be used.

The ROCK inhibitor used in the present invention may be Y-27632, which is a compound represented by the following formula I or a salt (e.g., a dihydrochloride) thereof. Y-27632 may be added as the form of a hydrate.

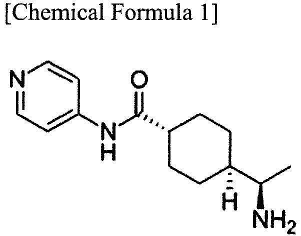

The concentration of Y-27632 in a liquid culture medium is not limited and is, for example, from 80 to 120 nM (in particular, 100 nM), from 400 to 600 nM (in particular, 500 nM), from 600 to 900 nM (in particular, 750 nM), from 0.8 to 1.2 µM (in particular, 1 µM), from 1.6 to 2.4 µM (in particular, 2 µM), from 2.4 to 3.6 µM (in particular, 3 µM), from 3.2 to 4.8 µM (in particular, 4 µM), from 4 to 6 µM (in particular, 5 µM), from 4.8 to 7.2 µM (in particular, 6 µM), µM (in particular, 7 µM), from 6.4 to 9.6 µM (in particular, 8 µM), from 7.2 to 10.8 µM (in particular, 9 µM), from 8 to 12 µM (in particular, 10 µM), from 12 to 18 µM(in particular, 15 µM), from 16 to 24 µM (in particular, 20 µM), from 20 to 30 µM (in particular, 25 µM), from 24 to 36 µM (in particular, 30 µM), from 32 to 48 µM (in particular, 40 µM), or from 40 to 60 µM (in particular, 50 µM), more preferably from 8 to 12 µM (in particular, 10 µM).

### <4. Lipids>

A lipid according to an embodiment of the present invention may have an ability to bind to albumin. In this section <4. Lipids>, unless otherwise indicated, the "lipid" refers to a lipid having an ability to bind to albumin. Specific examples of the lipid include several forms of lipids: lipids that form a complex with a protein (e.g., serum albumin); lipids isolated from serum albumin; and lipids that are produced in microorganisms or produced through chemical synthesis and that have an ability to bind to albumin. Note that the "ability to bind to albumin" refers to a characteristic in which the lipid can form a complex with albumin by means of chemical and/or physical interaction.

Examples of animals from which the lipid is derived may include: mammals such as rodents (e.g., a mouse, rat, hamster), primates (e.g., a human, gorilla, chimpanzee), and domestic animals and pets (e.g., a dog, cat, rabbit, cow, horse, sheep, goat, pig). Preferred is a cow or human. It is also preferable that the lipid is derived from the same organism species as of cells to be cultured. In addition, the lipid may be artificially synthesized.

The lipid may be added, as a serum containing the lipid, to a liquid culture medium. In addition, the lipid may be added, as serum albumin containing the lipid (lipid-containing serum albumin), to a liquid culture medium. As the lipid-containing serum albumin, it is possible to use a lipid-protein (of serum albumin) complex. Among various types of the lipid-containing serum albumin, particularly preferred are those having an increased content of lipid. Examples of the lipid-containing serum albumin include commercially available AlbuMAX^{™}. Specific examples are AlbuMAX^{™} I or AlbuMAX^{™} II (both come from Life Technologies Japan Ltd.).

The lipid had no particular limitation as long as the lipid is derived from serum or can bind to serum albumin. Also, the lipid may vary depending on a source organism. Thus, the lipid may be a free fatty acid, phospholipid (e.g., a glycerophospholipid, sphingophospholipid, lysophosphatidylcholine), neutral fat, or cholesterol. The lipid, for example, contains at least one, preferably all of lipids selected from the group consisting of lysophosphatidylcholine, triacylglyceride, phosphatidylcholine, phosphatidic acid, cholesterol, and sphingomyelin. More preferably, the lipid used in the present invention contains, as a total lipid weight basis, 20 to 80 wt% (more preferably 40 to 70 wt%) of a free fatty acid, 5 to 50 wt% (more preferably 10 to 30 wt%) of lysophosphatidylcholine, 5 to 45 wt% (more preferably 10 to 30 wt%) of a triacylglyceride, and 2 to 25 wt% (more preferably 5 to 15 wt%) of phosphatidylcholine, at least. Still more preferably, the lipid further contains at least one selected from the group consisting of 1 to 10 wt% (more preferably 1 to 6 wt%) of phosphatidic acid, 0.1 to 3 wt% (more preferably 0.5 to 2 wt%) of cholesterol, and 0.1 to 3 wt% (more preferably 0.5 to 2 wt%) of sphingomyelin. The lipid-containing serum albumin containing such a composition of lipid may be suitably used for the method of the present invention. Note that according to Non-Patent Literature 2, AlbuMAX^{™} II contains, as a total lipid weight basis, about 54 wt% of a free fatty acid, about 17 wt% of lysophosphatidylcholine, about 15 wt% of a triacylglyceride, about 8 wt% of phosphatidylcholine, about 3 wt% of phosphatidic acid, about 1 wt% of cholesterol, and about 1 wt% of sphingomyelin.

The lipids isolated from serum albumin may be preferably used in the present invention. Examples of a method for isolating the lipids from the serum albumin include, but are not particularly limited to, a method comprising subjecting a lipid-containing serum albumin complex to protease (e.g. trypsin) treatment to recover the lipids. The isolated lipids may be used in the present invention while the composition of the lipids contained in the serum albumin is kept substantially the same. Alternatively, some of the lipids may be enriched and used.

The lipids may be included in a serum replacement commercially available. In this case, the lipids may be added, to a liquid culture medium, as one component included in the serum replacement and then used. The serum replacement is a reagent used as a serum (e.g., FBS) replacement so as to maintain an undifferentiated state of ES cells or iPS cells and culture them. Examples of the serum replacement include KNOCKOUT^{™} SR (KnockOut^{™} Serum Replacement (KSR); Life Technologies Japan Ltd.), StemSure (a registered trademark) Serum Replacement (SSR; Wako Pure Chemical Industries, Ltd.), and N2 supplement (Wako Pure Chemical Industries, Ltd.). The above KSR contains AlbuMAX^{™}, specifically, AlbuMAX^{™} I or AlbuMAX^{™} II.

Generally speaking, the concentration of albumin included in serum is about 50 mg/ml. It reasonably assumes that the KSR contains a similar concentration of serum albumin. According to Non-Patent Literature 2, the serum albumin included in the KSR is AlbuMAX^{™}. In addition, Non-Patent Literature 2 discloses that the amount of lipids included in 100 mg of AlbuMAX^{™} is 0.65 mg. Thus, a liquid culture medium containing a final concentration of 1% (v/v) KSR, for example, contains 0.5 mg/ml of AlbuMAX^{™}, and, accordingly, the concentration of lipids included is 0.00325 mg/ml. For example, a liquid culture medium containing a final concentration of 2 mg/ml AlbuMAX^{™} contains 0.013 mg/mL of lipids. Hence, the content of lipids in a liquid culture medium used for suspension culture according to the present invention has no particular limitation and may be from 0.00325 to 0.065 mg/ml, preferably from 0.00325 to 0.0325 mg/ml, more preferably from 0.00325 to 0.01625 mg/ml, and still more preferably from 0.00325 to 0.013 mg/ml. The concentration of lipids may be determined by appropriate analysis protocols (e.g., analysis protocols using liquid chromatography, gas chromatography, and other means).

The lipid may be present in a liquid culture medium under conditions in which the lipid is free of albumin. A separately prepared lipid and albumin may be added to a liquid culture medium. Preferably, examples of the origin of albumin added include, but are not particularly limited to, human or bovine albumin. That is, a method according to an embodiment of the present invention may comprise a step of adding an albumin-free lipid to prepare a liquid culture medium. In addition, lipid-free serum albumin may be employed. It may also be possible to use albumin as prepared by expressing the albumin in *E. coli* or animal cells using a gene recombinant technology. Proteins or additives, which replace albumin, for example, amphiphilic substances (e.g., a surfactant) may also be added.

### <5. Lysophospholipid>

A lysophospholipid is used as a lipid according to the present invention., wherein the liquid culture medium comprises the lysophospholipid in an amount of more than 0.0064 mg/mL and 100 mg/
mL or less and wherein the lysophospholipid is at least one of lysophosphatidic acid and sphingosine-1- phosphoric acid as recited in the appended claims.

The lysophospholipid is a general term for a phospholipid having an aliphatic group (e.g., a medium or long chain aliphatic group). The lysophospholipid may have a glycerol or sphingosine backbone. Examples of the lysophospholipid include lysophosphatidic acid (LPA), sphingosine-1-phosphoric acid (S1P), lysophosphatidylcholine (LPC), lysophosphatidylserine (LPS), lysophosphatidylinositol (LPI), lysophosphatidylglycerol (LPG), lysophosphatidylthreonine (LPT), and lysophosphatidyl ethanolamine (LPE). It may be a mixture containing a plurality of lysophospholipids. These lysophospholipids may be in any form (e.g., a salt). The lysophospholipid is preferably a lysophospholipid other than LPC. More preferred are, for example, LPA and S1P, which are a lysophospholipid having a non-substituted phosphate group as a polar head group. When the lysophospholipid contains an acyl group, the number of carbons and the degree of unsaturation of the acyl group have no particular limitation. The number of carbons and the degree of unsaturation of the acyl group may depend on a source organism. Usually, the acyl group has 16 to 24 carbons and the degree of unsaturation ranges from 0 to 6. More preferably, the number of carbons : the degree of unsaturation in the acyl group is 16:0, 16:1, 18:0, 18:1, 18:2, 18:3, 20:0, 20:1, 20:2, 20:3, 20:4, 20:5, 22:0, 22:1, 22:2, 22:3, 22:4, 22:5, or, 22:6. In addition, the lysophospholipid with a glycerol backbone may be either 1-acyl lysophospholipid or 2-acyl lysophospholipid. 1-acyl lysophospholipid is preferable.

The source organism of the lysophospholipid may be selected from those of a similar source organism range described in the section <4. Lipids>. In addition, the lysophospholipid may be artificially prepared.

The lysophospholipid may be added to a liquid culture medium as a lysophospholipid-containing composition. Examples of the composition include a mixture of a lysophospholipid and a protein.

The lysophospholipid that is not in complex with a protein may be used in a culture. A lysophospholipid not in the form of a mixture with a protein, preferably a lysophospholipid in a substantially purified form, may be used as a material for preparation of a liquid culture medium. In this case, it is easy to adjust the additive amount of the lysophospholipid to a preferable range, which is preferable. Such a lysophospholipid may be isolated from a source organism or may be artificially prepared.

At the start of suspension culture, the concentration of the lysophospholipid in a liquid culture medium may be adjusted appropriately. For example, the concentration may be 0.00128 µg/mL or more, preferably 0.0064 µg/mL or more, preferably more than 0.0064 µg/mL, preferably 0.032 µg/mL or more, preferably 0.064 µg/mL or more, preferably 0.16 µg/mL or more, preferably 0.2 µg/mL or more, preferably 0.3 µg/mL or more, preferably 0.4 µg/mL or more, or preferably 0.5 µg/mL or more. In this case, aggregation of cells can be moderately suppressed to form cell aggregates with a substantially uniform size. For example, the concentration may be 1000 µg/mL or less, preferably 200 µg/mL or less, preferably 150 µg/mL or less, more preferably 100 µg/mL or less, more preferably 90 µg/mL or less, more preferably 80 µg/mL or less, more preferably 70 µg/mL or less, more preferably 60 µg/mL or less, more preferably 50 µg/mL or less, more preferably 40 µg/mL or less, more preferably 30 µg/mL or less, more preferably 20 µg/mL or less, more preferably 10 µg/mL or less, more preferably 9 µg/mL or less, more preferably 8 µg/mL or less, more preferably 7 µg/mL or less, more preferably 6 µg/mL or less, more preferably 5 µg/mL or less, more preferably 4 µg/mL or less, more preferably 3 µg/mL or less, more preferably 2 µg/mL or less, more preferably 1 µg/mL or less, more preferably 0.9 µg/mL or less, more preferably 0.8 µg/mL or less, more preferably 0.7 µg/mL or less, and more preferably 0.6 µg/mL or less. In the above cases, spherical cell aggregates with the above-mentioned suitable size may be formed. A plurality of kinds of lysophospholipids may be added, as components, to a liquid culture medium. In this case, it is preferable to set the concentration of each lysophospholipid to within the above range. It is more preferable to set the total concentration of the lysophospholipids to within the above range. Note that the amount of the above lysophospholipid refers to the amount of a lysophospholipid added when a liquid culture medium is prepared (provided that when a lysophospholipid is generated through an enzymatic reaction in a liquid culture medium as described below, the amount of interest includes the amount of a lysophospholipid generated by this reaction). The amount of interest does not include the amount of lysophospholipids produced by cultured cells. The concentration of the lysophospholipid may be determined by appropriate analysis protocols (e.g., analysis protocols using liquid chromatography, gas chromatography, and other means). More preferably, the concentration by weight of the lysophospholipid determined in terms of 2S-amino-1-(dihydrogen phosphate)-4E-octadecene-1,3R-diol (with a molecular weight of 379.5) is within the above range.

The lysophospholipid may be lysophosphatidic acid. In this case, at the start of suspension culture, the concentration of the lysophosphatidic acid in a liquid culture medium may be adjusted appropriately. For example, the concentration is 0.00128 µg/mL or more or 0.00279 µM or more, preferably 0.0064 µg/mL or more or 0.0140 µM or more, preferably more than 0.0064 µg/mL or more than 0.0140 µM, preferably 0.032 µg/mL or more or 0.0698 µM or more, preferably 0.064 µg/mL or more or 0.140 µM or more, preferably 0.16 µg/mL or more or 0.349 µM or more, preferably 0.2 µg/mL or more or 0.436 µM or more, preferably 0.3 µg/mL or more or 0.654 µM or more, preferably 0.4 µg/mL or more or 0.872 µM or more, and preferably 0.5 µg/mL or more or 1.09 µM or more. In the above cases, aggregation of cells may be moderately suppressed to form cell aggregates with a substantially uniform size. For example, the concentration may be 1000 µg/mL or less or 2180 µM or less, preferably 200 µg/mL or less or 436 µM or less, preferably 150 µg/mL or less or 327 µM or less, more preferably 100 µg/mL or less or 218 µM or less, more preferably 90 µg/mL or less or 196 µM or less, more preferably 80 µg/mL or less or 174 µM or less, more preferably 70 µg/mL or less or 153 µM or less, more preferably 60 µg/mL or less or 131 µM or less, more preferably 50 µg/mL or less or 109 µM or less, more preferably 40 µg/mL or less or 87.2 µM or less, more preferably 30 µg/mL or less or 65.4 µM or less, more preferably 20 µg/mL or less or 43.6 µM or less, more preferably 10 µg/mL or less or 21.8 µM or less, more preferably 9 µg/mL or less or 19.6 µM or less, more preferably 8 µg/mL or less or 17.4 µM or less, more preferably 7 µg/mL or less or 15.3 µM or less, more preferably 6 µg/mL or less or 13.1 µM or less, more preferably 5 µg/mL or less or 10.9 µM or less, more preferably 4 µg/mL or less or 8.72 µM or less, more preferably 3 µg/mL or less or 6.54 µM or less, more preferably 2 µg/mL or less or 4.36 µM or less, more preferably 1 µg/mL or less or 2.18 µM or less, more preferably 0.9 µg/mL or less or 1.96 µM or less, more preferably 0.8 µg/mL or less or 1.74 µM or less, more preferably 0.7 µg/mL or less or 1.53 µM or less, and more preferably 0.6 µg/mL or less or 1.31 µM or less. In the above cases, spherical cell aggregates with the above-mentioned suitable size may be formed. Note that the amount of the above lysophosphatidic acid refers to the amount of a lysophosphatidic acid added when a liquid culture medium is prepared (provided that when a lysophosphatidic acid is generated through an enzymatic reaction in a liquid culture medium as described below, the amount of interest includes the amount of a lysophosphatidic acid generated by this reaction). The amount of interest does not include the amount of lysophosphatidic acid produced by cultured cells. The concentration by weight of the above lysophosphatidic acid refers to the concentration by weight in terms of a sodium salt thereof (1-O-9Z-octadecenoyl-sn-glyceryl-3-phosphoric acid, sodium salt; with a molecular weight of 458.5).

The lysophospholipid may be sphingosine-1-phosphoric acid. In this case, at the start of suspension culture, the concentration of the sphingosine-1-phosphoric acid in a liquid culture medium may be adjusted appropriately. For example, the concentration is 0.00128 µg/mL or more or 0.00337 µM or more, preferably 0.0064 µg/mL or more or 0.0169 µM or more, preferably more than 0.0064 µg/mL or more than 0.0169 µM, preferably 0.032 µg/mL or more or 0.0843 µM or more, preferably 0.064 µg/mL or more or 0.169 µM or more, preferably 0.16 µg/mL or more or 0.422 µM or more, preferably 0.2 µg/mL or more or 0.527 µM or more, preferably 0.3 µg/mL or more or 0.791 µM or more, preferably 0.4 µg/mL or more or 1.05 µM or more, and preferably 0.5 µg/mL or more or 1.32 µM or more. In the above cases, aggregation of cells can be moderately suppressed to form cell aggregates with a substantially uniform size. For example, the concentration is 1000 µg/mL or less or 2640 µM or less, preferably 200 µg/mL or less or 527 µM or less, and preferably 150 µg/mL or less or 395 µM or less, more preferably 100 µg/mL or less or 264 µM or less, more preferably 90 µg/mL or less or 237 µM or less, more preferably 80 µg/mL or less or 211 µM or less, more preferably 70 µg/mL or less or 184 µM or less, more preferably 60 µg/mL or less or 158 µM or less, more preferably 50 µg/mL or less or 132 µM or less, more preferably 40 µg/mL or less or 105 µM or less, more preferably 30 µg/mL or less or 79.1 µM or less, more preferably 20 µg/mL or less or 52.7 µM or less, more preferably 10 µg/mL or less or 26.4 µM or less, more preferably 9 µg/mL or less or 23.7 µM or less, more preferably 8 µg/mL or less or 21.1 µM or less, more preferably 7 µg/mL or less or 18.4 µM or less, more preferably 6 µg/mL or less or 15.8 µM or less, more preferably 5 µg/mL or less or 13.2 µM or less, more preferably 4 µg/mL or less or 10.5 µM or less, more preferably 3 µg/mL or less or 7.91 µM or less, more preferably 2 µg/mL or less or 5.27 µM or less, more preferably 1 µg/mL or less or 2.64 µM or less, more preferably 0.9 µg/mL or less or 2.37 µM or less, more preferably 0.8 µg/mL or less or 2.11 µM or less, more preferably 0.7 µg/mL or less or 1.84 µM or less, and more preferably 0.6 µg/mL or less or 1.58 µM or less. In the above cases, spherical cell aggregates with the above-mentioned suitable size may be formed. Note that the amount of the above sphingosine-1-phosphoric acid refers to the amount of a sphingosine-1-phosphoric acid added when a liquid culture medium is prepared (provided that when sphingosine-1-phosphoric acid is generated through an enzymatic reaction in a liquid culture medium as described below, the amount of interest includes the amount of sphingosine-1-phosphoric acid generated by the above reaction). The amount of interest does not include the amount of sphingosine-1-phosphoric acid produced by cultured cells. Preferably, the concentration by weight of the sphingosine-1-phosphoric acid refers to the concentration by weight determined in terms of a free form thereof (2S-amino-1-(dihydrogen phosphate)-4E-octadecene-1,3R-diol; with a molecular weight of 379.5).

A plurality of the lysophospholipids may be mixed and used. Regarding the mixed ratio, examples of the ratio by weight of LPA to S1P include, but are not particularly limited to, 1:1 to 80000 or 1 to 80000:1. Specific examples of the ratio that can be used include 1:0.5 to 1.5 (e.g., 1:1), 1:2.5 to 7.5 (e.g., 1:5), 1:13 to 38 (e.g., 1:25), 1:63 to 190 (e.g., 1:125), 1:78 to 230 (e.g., 1:156.25), 1:310 to 940 (e.g., 1:625), 1:1600 to 4700 (e.g., 1:3125), 1:7800 to 23000 (e.g., 1:15625), 1:39000 to 120000 (e.g., 1:78125), 2.5 to 7.5:1 (e.g., 5:1), 13 to 38:1 (e.g., 25:1), 63 to 190:1 (e.g., 125:1), 78 to 230:1 (e.g., 156.25:1), 78 to 230:5 (e.g., 156.25:5), 78 to 230:25 (e.g., 156.25:25), 78 to 230:125 (e.g., 156.25:125), 310 to 940:1 (e.g., 625:1), 310 to 940:156.25 (e.g., 625:156.25), 1600 to 4700:1 (e.g., 3125:1), 1600 to 4700:156.25 (e.g., 3125:156.25), 7800 to 23000:1 (e.g., 15625:1), 7800 to 23000:156.25 (e.g., 15625:156.25), 39000 to 120000:1 (e.g., 78125:1), and 39000 to 120000:156.25 (e.g., 78125:156.25). LPA is preferably determined in terms of a sodium salt thereof (1-O-9Z-octadecenoyl-sn-glyceryl-3-phosphoric acid, sodium salt; with a molecular weight of 458.5) and S1P is determined in terms of a free form thereof (2S-amino-1-(dihydrogen phosphate)-4E-octadecene-1,3R-diol; with a molecular weight of 379.5) to calculate the weight ratio.

Lysophospholipids including lysophosphatidylcholine (LPC), lysophosphatidylserine (LPS), lysophosphatidylinositol (LPI), lysophosphatidylglycerol (LPG), lysophosphatidylthreonine (LPT), or lysophosphatidyl ethanolamine (LPE) may be used in the above concentration or mixed ratio in addition to lysophosphatidic acid (LPA) and sphingosine-1-phosphoric acid (S1P).

The method of the present invention may comprise a step of carrying out an enzymatic reaction to produce from a lipid the above lysophospholipid. Examples of the enzymatic reaction may include a hydrolase reaction and a kinase reaction.

Examples of the hydrolase reaction include hydrolysis catalyzed by a hydrolase using the above phospholipid as a substrate. Preferable examples of the hydrolase used in the present invention include, but are not particularly limited to, phospholipases and lysophospholipases. Preferable examples of the phospholipases and lysophospholipases include, but are not particularly limited to, phospholipase A1, phospholipase A2, and lysophospholipase D (Autotaxin). Phospholipase A1 hydrolyzes an ester bond at sn-1 position of a glycerophospholipid. Phospholipase A2 hydrolyzes an ester bond at sn-2 position of a glycerophospholipid. Autotaxin is a hydrolase that hydrolyzes a phosphoester bond between a phosphate group and a substituent of a phospholipid or lysophospholipid to generate an unsubstituted phosphate group as a polar head group. Autotaxin can hydrolyze LPC to produce LPA and choline. Phospholipase A1 or phospholipase A2 and Autotaxin hydrolyze a glycerophospholipid as a substrate to produce hydrolysates (e.g., LPA), which are preferably used as a lysophospholipid in the present invention. For example, LPC, LPS, LPI, LPG, LPT, or LPE, is subject to the above Autotaxin treatment to give hydrolysates (e.g., LPA) that may be suitably used in the present invention.

In the present invention, S1P that is prepared from the above sphingo lipid is used as a lysophospholipid. The preparation method has no particular limitation. For example, a sphingosine is phosphorylated in the presence of a kinase such as a sphingokinase to give a phosphorylated product (e.g., S1P), which may be suitably used as a lysophospholipid.

The method of the present invention may further comprise a step of adding the above lysophospholipid to prepare a liquid culture medium. At this time, it is preferable to add the above lysophospholipid in a form not in complex with a protein because it is easy to control the additive amount of the lysophospholipid.

In the present disclosure, a cell aggregation inhibition kit comprising the lysophospholipid (preferably comprising either LPA or S1P), the hydrolysate of the lipid, a mixture of the lipid and the hydrolase, a mixture of the sphingo lipid and the kinase, or a liquid culture medium containing any of these materials, may be suitably used.

### <6. Cell Aggregation Inhibitor>

A cell aggregation inhibitor according to the present invention contains the lipid as recited in the appended claims and is used to inhibit aggregation of cells in a suspension culture system to form cell aggregates with a substantially uniform size.

In the present invention, the cell aggregation inhibitor may be in any form. The cell aggregation inhibitor may be the above lipid itself or may be a composition produced by combining the above lipid and another component. The form of the composition is not particularly limited. The composition may be, for example, a liquid culture medium used for suspension culture or may be an additive composition mixed when a liquid culture medium is prepared.

In the present invention, preferable embodiments of the cell aggregation inhibitor may be the liquid culture medium or a buffer (e.g., a phosphate buffer) containing the lysophospholipid(s) in the above-described concentration or ratio.

In the present invention, other preferable embodiments of the cell aggregation inhibitor may be a liquid composition containing the lysophospholipid(s) in a liquid medium. The liquid composition is an additive mixed when a liquid culture medium for suspension culture is prepared. Preferably, the liquid composition is prepared such that the final concentration of the lysophospholipid in a liquid culture medium prepared is within the above described concentration. The concentration of the lysophospholipid in the liquid composition before mixed with the cells of interest has no particular limitation. Preferably, the concentration of the lysophospholipid is 1 or more, preferably 10 or more, more preferably 100 or more, still more preferably 1000 or more, and still more preferably 10000 or more times the above-mentioned concentration specified as a preferable concentration during suspension culture.

The cell aggregation inhibitor may also contain, as additives, an enzyme (in particular, a hydrolase, kinase), antibiotic, kinase inhibitor, buffer, thickener, colorant, stabilizer, surfactant, emulsifier, preservative, preserving agent, or antioxidant. The enzyme is not particularly limited and a hydrolase or a kinase, for example, may be used. The hydrolase or kinase is as described above. Examples of the antibiotics that can be used include, but are not particularly limited to, penicillin, streptomycin, and amphotericin B. Preferable examples of the kinase inhibitor include, but are not particularly limited to, ROCK inhibitors. Preferable examples of the ROCK inhibitors include, but are not particularly limited to, Y-27632. It is preferable that the cell aggregation inhibitor according to the present invention contains a ROCK inhibitor in the above-described concentration as a final concentration in a liquid culture medium. It is the most preferable that the final concentration of the ROCK inhibitor in a liquid culture medium is 10 µM. Examples of the buffer include a phosphate buffer, tris-hydrochloric acid buffer, and glycine buffer. Examples of the thickener include gelatin and polysaccharides. Examples of the colorant include Phenol Red. Examples of the stabilizer include albumin, dextran, methyl cellulose, and gelatin. Examples of the surfactant include cholesterol, an alkyl glycoside, alkyl polyglycoside, alkyl monoglyceride ether, glucoside, maltoside, neopentyl glycol series, polyoxyethylene glycol series, thioglucoside, thiomaltoside, peptide, saponin, phospholipid, sorbitan fatty acid ester, and fatty acid diethanolamide. Examples of the emulsifier include a glycerin fatty acid ester, sorbitan fatty acid ester, propylene glycol fatty acid ester, and sucrose fatty acid ester. Examples of the preservative include aminoethyl sulfonic acid, benzoic acid, sodium benzoate, ethanol, sodium edetate, agar, dl-camphor, citric acid, sodium citrate, salicylic acid, sodium salicylate, phenyl salicylate, dibutylhydroxy toluene, sorbic acid, potassium sorbate, nitrogen, dehydro acetic acid, sodium dehydroacetate, 2-naphthol, white soft sugar, honey, paraoxy isobutyl benzoate, paraoxy isopropyl benzoate, paraoxy ethyl benzoate, paraoxy butyl benzoate, paraoxy propyl benzoate, paraoxy methyl benzoate, 1-menthol, and eucalyptus oil. Examples of the preserving agent include benzoic acid, sodium benzoate, ethanol, sodium edetate, dried sodium sulfite, citric acid, glycerin, salicylic acid, sodium salicylate, dibutylhydroxy toluene, D-sorbitol, sorbic acid, potassium sorbate, sodium dehydroacetate, paraoxy isobutyl benzoate, paraoxy isopropyl benzoate, paraoxy ethyl benzoate, paraoxy butyl benzoate, paraoxy propyl benzoate, paraoxy methyl benzoate, propylene glycol, and phosphoric acid. Examples of the antioxidant include citric acid, citric acid derivatives, vitamin C and derivatives thereof, lycopene, vitamin A, carotenoids, vitamin B and derivatives thereof, flavonoids, polyphenols, glutathione, selenium, sodium thiosulfate, vitamin E and derivatives thereof, α-lipoic acid and derivatives thereof, pycnogenol, flavangenol, super oxide dismutase (SOD), glutathione peroxidase, glutathione-S-transferase, glutathione reductase, catalase, ascorbic acid peroxidase, and mixtures thereof.

The cell aggregation inhibitor may contain a growth factor. Preferably, the cell aggregation inhibitor contains at least one of FGF2 and TGF-β1.

### <7. Suspension Culture>

In an embodiment of the present invention, cells are cultured in suspension in a liquid culture medium to form cell aggregates. In the present invention, it is preferable to culture cells in suspension under conditions in which, assuming that the prescribed lipid of the present invention is not present in the liquid culture medium, cell aggregates with more than the above-described suitable size would be formed.

Cultureware used for suspension cell culture is preferably a container on which cells adhere less to an inner surface thereof. Examples of such a container include plates, the surface of which is subjected to hydrophilic treatment with a biocompatible substance. Examples of the cultureware that may be used include, but are not particularly limited to, Nunclon^{™} Sphera (Thermo Fisher Scientific Inc.).

Examples of the shape of the cultureware include, but are not particularly limited to, a dish, flask, well, bag, and spinner flask shape.

The suspension culture may be static culture. Also, the culture may be performed under conditions in which a liquid culture medium flows. Preferably, the culture may be performed under conditions in which a liquid culture medium flows. When the culture is performed under conditions in which a liquid culture medium flows, the culture is preferably performed under the conditions so as to promote cell aggregation. Examples of this culture include: culture under conditions in which a liquid culture medium flows such that cells are concentrated on a spot due to stress (e.g., centrifugal force, centripetal force) caused by, for example, a swirling and/or rocking flow; and culture under conditions in which a liquid culture medium flows due to a linear back and forth movement. Particularly preferred is culture performed using a swirling and/or rocking flow.

The rotary shaking culture (i.e., culture under shaking) is carried out such that cultureware housing cells in a liquid culture medium is subject to rotary shaking substantially along the horizontal plane in a closed path (e.g., a circle, ellipse, flat circle, flat ellipse). The speed of rotation has no particular limitation and the upper limit is preferably 200 rpm, more preferably 150 rpm, still more preferably 120 rpm, still more preferably 115 rpm, still more preferably 110 rpm, still more preferably 105 rpm, still more preferably 100 rpm, still more preferably 95 rpm, and still more preferably 90 rpm. The lower limit is preferably 1 rpm, more preferably 10 rpm, still more preferably 50 rpm, still more preferably 60 rpm, still more preferably 70 rpm, still more preferably 80 rpm, and still more preferably 90 rpm. The shaking width during rotary shaking culture has no particular limitation and the lower limit is, for example, 1 mm, preferably 10 mm, more preferably 20 mm, and most preferably 25 mm. The upper limit of the shaking width is, for example, 200 nm, preferably 100 mm, more preferably 50 mm, still more preferably 30 mm, and most preferably 25 mm. The radius of rotation during rotary shaking culture has no particular limitation and is preferably set such that the shaking width is within the above-described range. The lower limit of the radius of rotation is, for example, 5 mm and preferably 10 mm. The upper limit of the radius of rotation is, for example, 100 mm and preferably 50 mm. Setting the rotary shaking culture condition to this range is preferable because it is easy to prepare cell aggregates with an appropriate size.

The rocking culture is carried out while a liquid culture medium flows and is mixed by rocking. The rocking culture is carried out such that cultureware housing cells in a liquid culture medium is rocked in the direction of a plane substantially vertical to the horizontal plane. The speed of rocking has no particular limitation and is, for example, from 2 to 50 times (one back and forth movement is counted as one time) per minute and preferably from 4 to 25 times per minute. The angle of rocking has no particular limitation and is, for example, from 0.1 to 20 degrees and preferably from 2 to 10 degrees. Setting the rocking culture condition to this range enables cell aggregates with an appropriate size to be produced, which is preferable.

Further, the culture may be mixed by movement in which the above rotary shaking and rocking are combined.

Culture using spinner flask-shaped cultureware in which mixing blades are placed may be carried out. During this culture, the liquid culture medium is mixed by the mixing blades. The speed of rotation and the volume of culture medium are not particularly limited. When commercially available spinner flask-shaped cultureware is usedm, the culture medium volume recommended by the manufacturer may be suitably used. The speed of rotation has no particular limitation and may be, for example, 10 rpm or more and 100 rpm or less.

The seeding density (i.e., the cell density at the start of suspension culture) of cells cultured in suspension in a liquid culture medium may be adjusted appropriately. The lower limit is, for example, 0.01 × 10⁵ cells/ml, preferably 0.1 × 10⁵ cells/ml, and more preferably 1 × 10⁵ cells/ml. The upper limit of the seeding density is, for example, 20 × 10⁵ cells/ml and preferably 10 × 10⁵ cells/ml. When the seeding density is within this range, cell aggregates with an appropriate size are likely to be formed.

The volume of culture medium during suspension culture may be appropriately adjusted depending on cultureware used. When a 12-well plate (with a bottom area per well of 3.5 cm² in a flat view) is used, for example, the volume may be 0.5 ml/well or more and preferably 1.5 ml/well or more. More preferred is 1 ml/well. When a 6-well plate (with a bottom area per well of 9.6 cm² in a flat view) is used, for example, the volume may be 1.5 mL/well or more, preferably 2 mL/well or more, and more preferably 3 mL/well. The volume may be 6.0 mL/well or less, preferably 5 mL/well or less, and more preferably 4 mL/well or less. When a 125-mL Erlenmeyer flask (an Erlenmeyer flask with a volume of 125 mL) is used, for example, the volume may be 10 mL/flask or more, preferably 15 mL/flask or more, more preferably 20 mL/flask or more, still more preferably 25 mL/flask or more, still more preferably 20 mL/flask or more, still more preferably 25 mL/flask or more, and still more preferably 30 mL/flask or more. The volume may be 50 mL/flask or less, preferably 45 mL/flask or less, and more preferably 40 mL/flask or less. When a 500-mL Erlenmeyer flask (an Erlenmeyer flask with a volume of 500 mL) is used, for example, the volume may be 100 mL/flask or more, preferably 105 mL/flask or more, more preferably 110 mL/flask or more, still more preferably 115 mL/flask or more, and still more preferably 120 mL/flask or more. The volume may be 150 mL/flask or less, preferably 145 mL/flask or less, more preferably 140 mL/flask or less, still more preferably 135 mL/flask or less, still more preferably 130 mL/flask or less, and still more preferably 125 mL/flask or less. When a 1000-mL Erlenmeyer flask (an Erlenmeyer flask with a volume of 1000 mL) is used, for example, the volume may be 250 mL/flask or more, preferably 260 mL/flask or more, more preferably 270 mL/flask or more, still more preferably 280 mL/flask or more, and still more preferably 290 mL/flask or more. The volume may be 350 mL/flask or less, preferably 340 mL/flask or less, more preferably 330 mL/flask or less, still more preferably 320 mL/flask or less, and still more preferably 310 mL/flask or less. When a 2000-mL Erlenmeyer flask (an Erlenmeyer flask with a volume of 2000 mL) is used, for example, the volume may be 500 mL/flask or more, preferably 550 mL/flask or more, and more preferably 600 mL/flask or more. The volume may be 1000 mL/flask or less, preferably 900 mL/flask or less, more preferably 800 mL/flask or less, and still more preferably 700 mL/flask or less. When a 3000-mL Erlenmeyer flask (an Erlenmeyer flask with a volume of 3000 mL) is used, for example, the volume may be 1000 mL/flask or more, preferably 1100 mL/flask or more, more preferably 1200 mL/flask or more, still more preferably 1300 mL/flask or more, still more preferably 1400 mL/flask or more, and still more preferably 1500 mL/flask or more. The volume may be 2000 mL/flask or less, preferably 1900 mL/flask or less, more preferably 1800 mL/flask or less, still more preferably 1700 mL/flask or less, and still more preferably 1600 mL/flask or less. When a 2-L culture bag (a disposable culture bag with a volume of 2 L) is used, for example, the volume may be 100 mL/bag or more, preferably 200 mL/bag or more, more preferably 300 mL/bag or more, still more preferably 400 mL/bag or more, still more preferably 500 mL/bag or more, still more preferably 600 mL/bag or more, still more preferably 700 mL/bag or more, still more preferably 800 mL/bag or more, still more preferably 900 mL/bag or more, and still more preferably 1000 mL/bag or more. The volume may be 2000 mL/bag or less, preferably 1900 mL/bag or less, more preferably 1800 mL/bag or less, still more preferably 1700 mL/bag or less, still more preferably 1600 mL/bag or less, still more preferably 1500 mL/bag or less, still more preferably 1400 mL/bag or less, still more preferably 1300 mL/bag or less, still more preferably 1200 mL/bag or less, and still more preferably 1100 mL/bag or less. When a 10-L culture bag (a disposable culture bag with a volume of 10 L) is used, for example, the volume may be 500 mL/bag or more, preferably 1 L/bag or more, more preferably 2 L/bag or more, still more preferably 3 L/bag or more, still more preferably 4 L/bag or more, and still more preferably 5 L/bag or more. The volume may be 10 L/bag or less, preferably 9 L/bag or less, more preferably 8 L/bag or less, still more preferably 7 L/bag or less, and still more preferably 6 L/bag or less. When a 20-L culture bag (a disposable culture bag with a volume of 20 L) is used, for example, the volume may be 1 L/bag or more, preferably 2 L/bag or more, more preferably 3 L/bag or more, still more preferably 4 L/bag or more, still more preferably 5 L/bag or more, still more preferably 6 L/bag or more, still more preferably 7 L/bag or more, still more preferably 8 L/bag or more, still more preferably 9 L/bag or more, and still more preferably 10 L/bag or more. The volume may be 20 L/bag or less, preferably 19 L/bag or less, more preferably 18 L/bag or less, still more preferably 17 L/bag or less, still more preferably 16 L/bag or less, still more preferably 15 L/bag or less, still more preferably 14 L/bag or less, still more preferably 13 L/bag or less, still more preferably 12 L/bag or less, and still more preferably 11 L/bag or less. When a 50-L culture bag (a disposable culture bag with a volume of 50 L) is used, for example, the volume may be 1 L/bag or more, preferably 2 L/bag or more, more preferably 5 L/bag or more, still more preferably 10 L/bag or more, still more preferably 15 L/bag or more, still more preferably 20 L/bag or more, and still more preferably 25 L/bag or more. The volume may be 50 L/bag or less, preferably 45 L/bag or less, more preferably 40 L/bag or less, still more preferably 35 L/bag or less, and still more preferably 30 L/bag or less. When the volume of culture medium is within these ranges, cell aggregates with an appropriate size are likely to be formed.

The volume of cultureware used has no particular limitation and may be suitably selected. The area of the bottom of a portion housing a liquid culture medium may be determined in a flat view. The lower limit of the bottom area of the cultureware used may be, for example, 0.32 cm², preferably 0.65 cm², more preferably 0.65 cm², still more preferably 1.9 cm², and still more preferably 3.0 cm², 3.5 cm², 9.0 cm², or 9.6 cm². The upper limit of the bottom area of the cultureware used may be, for example, 1000 cm², preferably 500 cm², more preferably 300 cm², still more preferably 150 cm², still more preferably 75 cm², still more preferably 55 cm², still more preferably 25 cm², still more preferably 21 cm², still more preferably 9.6 cm², and still more preferably 3.5 cm².

Conditions (e.g., the temperature, culture period, CO₂ level) of cell suspension culture in the presence of the above lipid have no particular limitation. The culture temperature is preferably 20°C or higher and more preferably 35°C or higher and preferably 45°C or lower and more preferably 40°C or lower. Most preferred is 37°C. The culture period is preferably 0.5 hour or longer and more preferably 12 hours or longer and preferably 7 days or shorter, more preferably 72 hours or shorter, still more preferably 48 hours or shorter, and most preferably 24 hours or shorter. The CO₂ level during the culture is preferably 4% or higher and more preferably 4.5% or higher and preferably 10% or lower and more preferably 5.5% or lower. Most preferred is 5%. The suspension culture may be split. When the culture conditions are within these ranges, cell aggregates with an appropriate size are likely to be formed.

Cells used in suspension culture may be pre-cultured and maintained in accordance with a common procedure. The maintenance culture may be adherent culture in which cells are cultured in contact with a culture substrate (e.g., a support) or may be suspension culture in which cells are cultured in suspension in a culture medium. Cells under the maintenance culture are detached from a culture substrate or are detached from one another by using the above-mentioned detachment agent. The resulting cells are sufficiently dispersed and are then cultured in suspension. In order to disperse the cells, they are made to pass through a strainer and can be dispersed as single cells.

Cell aggregates that have been formed by suspension culture in the presence of the above lipid may be further cultured. Examples of a method for further culturing cell aggregates include a method comprising suspending and culturing cell aggregates in a liquid culture medium free of the kinase inhibitor. As the liquid culture medium used for this additional culture, substantially the same liquid culture medium except that it needs to be free of kinase inhibitor may be used. The conditions used for this additional culture may be substantially the same conditions as the above. In this additional culture, it is preferable that a culture medium should be changed in an appropriate frequency. The frequency of the medium change may vary depending on a type of the cells. The frequency of medium change operation may be preferably once or more per 5 days, more preferably once or more per 4 days, still more preferably once or more per 3 days, still more preferably once or more per 2 days, and most preferably once or more per day. This frequency of the medium change is particularly suitable when cell aggregates of pluripotent stem cells prepared in the present invention are cultured. The medium change procedure has no particular limitation. A preferable procedure may comprise: collecting all the volume of cell aggregate-containing culture medium into a centrifuge tube; subjecting the tube to centrifugation or allowing the tube to stand for 5 min; keeping precipitated cell aggregates and removing the rest supernatant and thereafter; adding a fresh liquid culture medium; gently dispersing the cell aggregates and thereafter; and returning the liquid culture medium containing the dispersed cell aggregates to cultureware (e.g., a plate), so that the cell aggregates can be cultured continuously. The culture period of the additional culture has no particular limitation and is preferably from 3 to 7 days.

The wording "step of adding the lysophospholipid to prepare the liquid culture medium" in the present invention refers to addition of the lysophospholipid to the liquid culture medium in the above-described concentration or ratio. The lysophospholipid may be added to the liquid culture medium and the isolated cells may then be mixed therewith. Alternatively, the isolated cells may be mixed with the liquid culture medium and the lysophospholipid may then be added. It is preferable that the lysophospholipid is added to the liquid culture medium and the isolated cells are then mixed therewith. When the lysophospholipid is added to the liquid culture medium, a stabilizer may be added. The stabilizer has no particular limitation as long as the substance can contribute to, for example, stabilization of the lysophospholipid in a liquid culture medium, maintenance of its activity, and prevention of adsorption on cultureware. A protein (e.g., albumin), emulsifier, surfactant, amphiphilic substance, or polysaccharide compound (e.g., heparin), for example, may be used. The "step of adding the lysophospholipid to prepare the liquid culture medium" may comprise a step of freezing the liquid culture medium containing the lysophospholipid (optionally containing the above-described stabilizer) and a step of thawing the liquid culture medium.

### Examples

The present invention is further described in detail by referring to the following Examples. However, they are just examples and do not restrict the present invention.

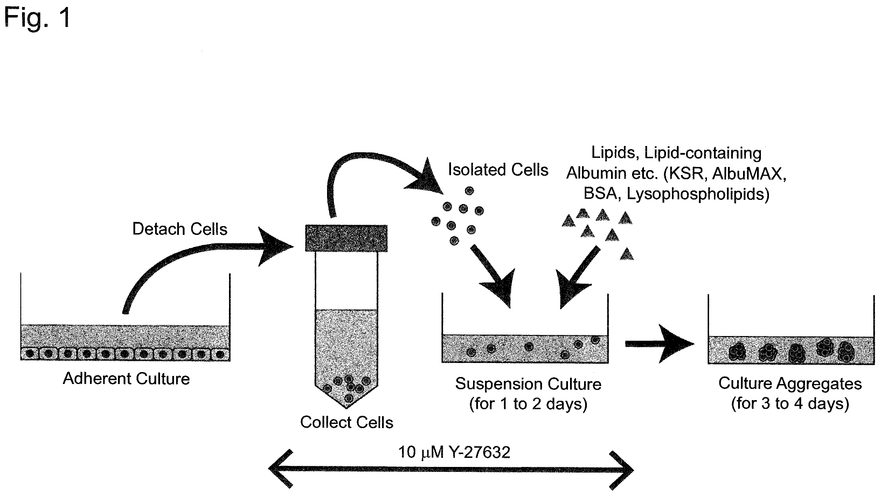

Figure 1 schematically illustrates an outline of a protocol for producing cell aggregates as described in the following Examples. First, human iPS cells were subject to adherent culture and were collected as isolated cells. Next, the cells were cultured in suspension in a liquid culture medium. Then, the cells were cultured to grow aggregates. In the following description, the first day when the suspension culture started was designated as "Day 0". The next day and later were each designated as "Day 1", "Day 2", "Day 3", "Day 4", or "Day 5". The suspension culture was performed until Day 2 and the aggregates were grown for 3 days (i.e., from the start of the suspension culture to Day 5).

### <Example 1: Maintenance Culture of Human iPS Cells>

TkDN4-M cell line (Non-Patent Literature 1) was used as human iPS cells. The human iPS cells were seeded on cell culture dishes coated with Matrigel (Corning, Inc.) or Vitronectin (Life Technologies Japan Ltd.). A culture medium, which was mTeSR1 (STEMCELL Technologies, Inc.) or Essential 8^{™} (Life Technologies Japan Ltd.), was used for maintenance culture. As a cell detachment agent at the time of cell passage, TrypLE Select (Life Technologies Japan Ltd.) was used when the cells were cultured on Matrigel; and 0.02% EDTA (ethylene diamine tetraacetic acid) solution or Accutase (Life Technologies Japan Ltd.) was used when the cells were cultured on Vitronectin. In addition, when the cells were seeded, Y-27632 (Wako Pure Chemical Industries, Ltd.) at a concentration of 10 µM was added to a culture medium. The culture medium was changed every day. For experiments, human iPS cells (the number of passage was 50 or less) were used.

### <Example 2: Formation of Aggregates of Human iPS cells>

Human iPS cells were treated with TrypLE Select or EDTA solution for 3 to 5 min and were detached. After dispersed as single cells, the cells were made to pass through a cell strainer with a pore size of 40 µm (Becton, Dickinson and Company). The resulting cells were suspended in Essential 8^{™} medium containing a final concentration of 10 µM Y-27632 (Wako Pure Chemical Industries, Ltd.). A portion thereof was then stained with trypan blue and the number of cells was counted. Suspensions containing 2 × 10⁵ cells per ml were prepared and different concentrations of KnockOut^{™} Serum Replacement (KSR; Life Technologies Japan Ltd.) were then added thereto. Each cell suspension was plated on a low-attachment 12-well plate (Nunc, Inc.) at a ratio of 1 ml/well. The cell-seeded plate was rotated along the horizontal plane on a rotary shaker (OPTIMA, Inc.) at a speed of 90 rpm in a circle with a shaking width (diameter) of 25 mm, so that the cells were subjected to rotary shaking culture. In this way, the cells were cultured in suspension under conditions at 5% CO₂ and 37°C until Day 2. At Day 2 after the start of culture, micrographs were obtained.

Subsequently, the culture medium was replaced by KSR (lipid)- and Y-27632-free Essential 8^{™} medium. The cells were then subjected to rotary shaking culture under the same conditions for 3 days (from the start of the suspension culture to Day 5). The culture medium was changed every day during that period. A medium change procedure involves: collecting all the volume of the culture medium containing cell aggregates; and letting them stand for about 5 min to precipitate the cell aggregates. Then, the supernatant was removed; a fresh medium was added; the cell aggregates were gently resuspended; and the cells were reseeded on a low-adherence 12-well plate.

Micrographs of cell aggregates were taken at Day 5 after the start of culture (Day 3 of the aggregation culture), and the size of each cell aggregate was analyzed by image-analyzing software (e.g., image J) to determine the diameter of each cell aggregate. After culturing, the aggregates were suspended and treated in TrypLE select (Life Technologies Japan Ltd.) for 10 min under conditions at 5% CO₂ and 37°C, and pipetted to disperse the aggregates into single cells. Then, the resulting cells were stained with trypan blue to count the number of cells.

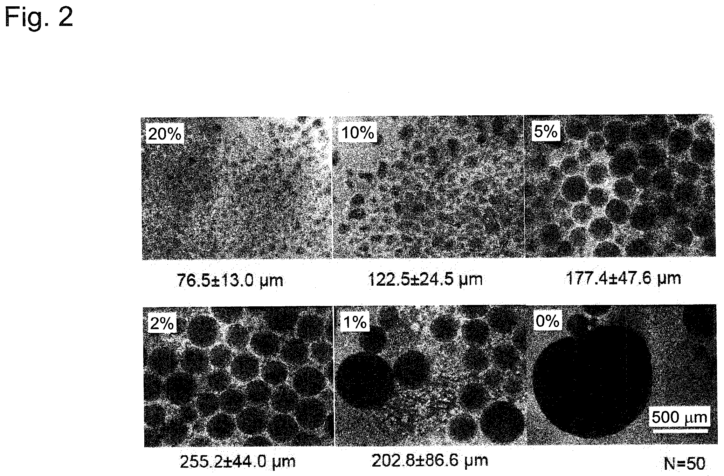

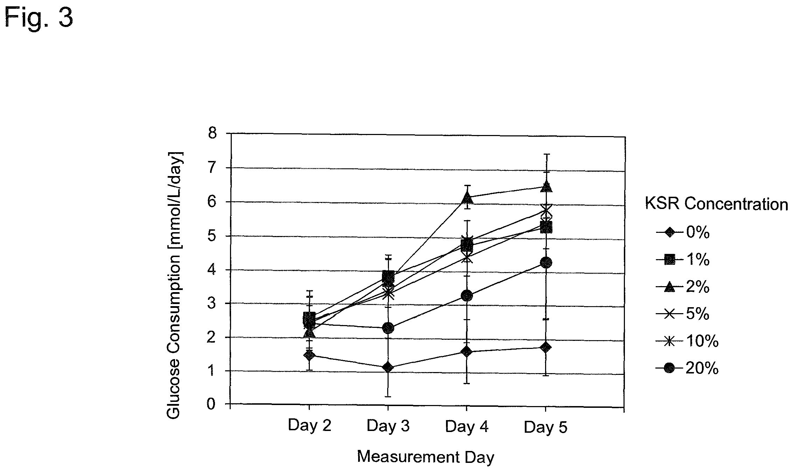

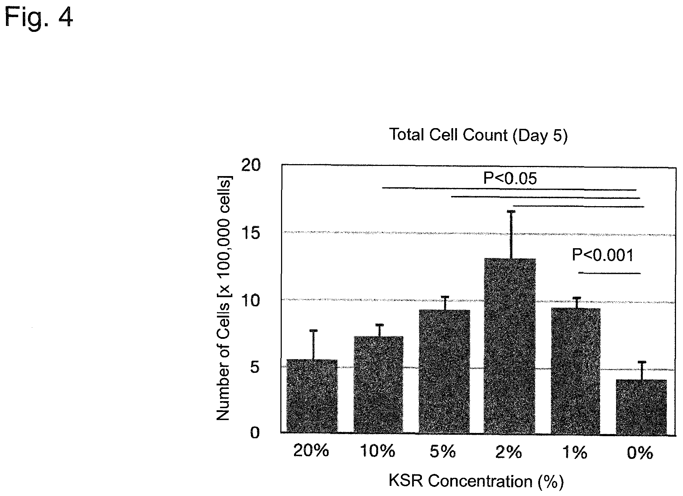

The above microscopic observation results demonstrated that under KSR-free conditions, large aggregates were formed, but when KSR was added, a large number of aggregates with a diameter of from 70 to 260 µm were formed (Figure 2). As the concentration of KSR added increased, the size of each aggregate became smaller. This revealed that some component included in KSR exerted an effect of inhibiting cell aggregation. In addition, with respect to the aggregates formed, the glucose consumption and the number of cells (at Day 5) at each KSR concentration were investigated. The results (Figures 3 and 4; n = 4 in both figures) demonstrated remarkable proliferation of cells when the concentration of KSR was from 1 to 10% (v/v) (when the content of lipid was from 0.00325 to 0.0325 mg/ml). This result demonstrated that efficient cell culture was made available when cells were cultured in such a condition that cell aggregates with a diameter of from 100 to 260 µm were formed. By contrast, excessive inhibition of cell aggregation clearly inhibited the proliferation of cells.

### <Example 3: KSR Contained Component Having Effect of Inhibiting Aggregation>

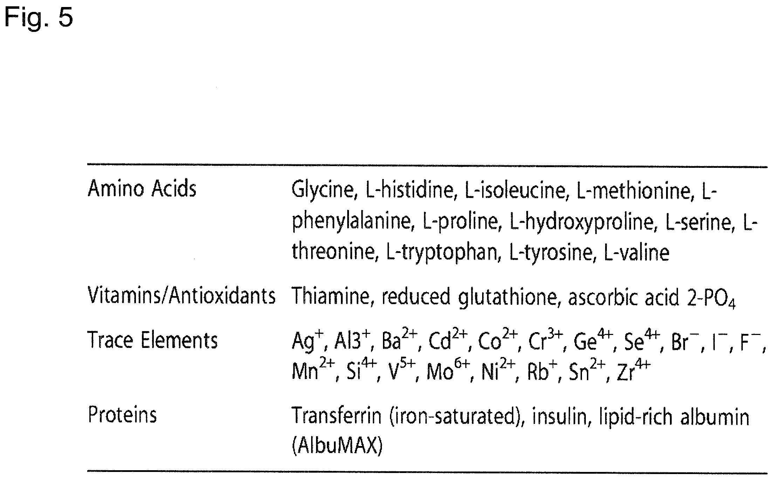

As components constituting KSR, components shown in Figure 5 have been reported (Non-Patent Literature 2).

It was examined which protein-based component (i.e., AlbuMAX^{™}, BSA, insulin, transferrin), which might seem to exert the effect of inhibiting aggregation, among the above components, exerted an effect of inhibiting the aggregation of human iPS cells when added.

Specifically, the following procedure was conducted: the same protocol as in Example 2 was repeated except that one of the following factors instead of KSR in Example 2 was each added, including various concentrations of lipid-rich albumin AlbuMAX^{™} II (Life Technologies Japan Ltd.), typical bovine serum albumin (BSA; Sigma-Aldrich Co. LLC.), insulin (Sigma-Aldrich Co. LLC.), and transferrin (Sigma-Aldrich Co. LLC.). That is, human iPS cells were subjected to rotary shaking culture (the cells were cultured in suspension in the presence of each factor for 2 days, followed by culture medium change and subsequent suspension culture for 3 days). Micrographs were obtained at Day 2 after the start of culture under conditions in which AlbuMAX^{™} II or BSA was added. Micrographs were obtained at Day 1 after the start of culture under conditions in which insulin or transferrin was added. The concentration of each factor added in Example 3, % (w/v), was represented in weight (g) per 100 ml of a liquid culture medium.

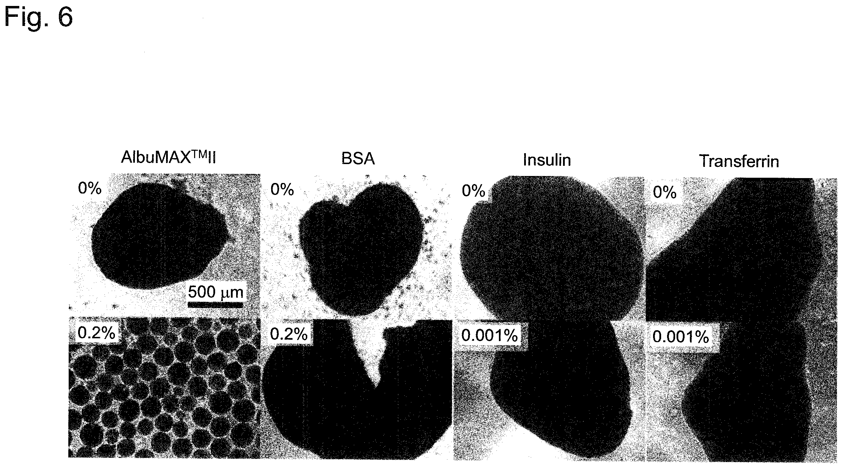

The results demonstrated that lipid-free insulin and transferrin caused large aggregates to be formed; and that an addition of 0.2% (w/v) AlbuMAX^{™} II (with a lipid content of 0.013 mg/ml) caused more marked inhibition of aggregation than an addition of 0.2% (w/v) BSA. This revealed that the lipids contained in AlbuMAX^{™} II, namely the lipids that can bind to albumin, inhibited the aggregation (Figure 6).

### <Example 4: Undifferentiated State of Human iPS Cells Forming Aggregates >

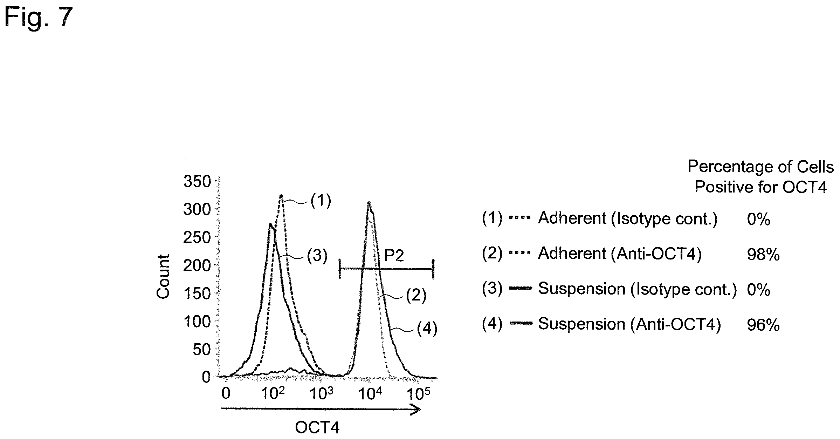

In Example 3, human iPS cells were subjected to culture conditions containing 0.2% (w/v) AlbuMAX^{™} II to form aggregates. These human iPS cell-derived aggregates were dispersed using Accutase (Life Technologies Japan Ltd.) and were washed with PBS (phosphate buffered saline). Next, the resulting cells were fixed with 4% PFA (paraformaldehyde) at room temperature for 20 min, then washed 3 times with PBS, and permeabilized with cold methanol at -20°C overnight. After washed 3 times with PBS, the cells were blocked with 3% FBS (fetal calf serum)/PBS and stained using a fluorescently labeled anti-OCT4 antibody (Cat. No. 653703, Biolegend, Inc.) at 4°C for 1 h. After washed once with 3% FBS (fetal calf serum)/PBS, the cells were made to pass through a cell strainer. The resulting cells were analyzed on FACSVerse (Becton, Dickinson and Company). The results demonstrated that 96% or more of human iPS cells that formed aggregates as well as human iPS cells during conventional adherent culture expressed OCT4, which is an undifferentiation marker; and that the human iPS cells forming aggregates remained undifferentiated (Figure 7).

### <Example 5: Effect of Phospholipid on Inhibiting Cell Aggregation>

How a phospholipid affected the formation of cell aggregates was analyzed.

### (Protocol)

Human iPS cells that had been cultured using the protocol of Example 1 were treated with TrypLE Select, EDTA solution, or Accutase for 3 to 5 min and were detached. After dispersed as single cells, the cells were made to pass through a cell strainer with a pore size of 40 µm (Becton, Dickinson and Company) to monodisperse as single cells. The resulting cells were suspended in Essential 8^{™} medium containing a final concentration of 10 µM Y-27632 (Wako Pure Chemical Industries, Ltd.). A portion thereof was then stained with trypan blue and the number of cells was counted. Suspensions containing 2 × 10⁵ cells per ml were prepared. To each cell suspension were added lipid-free bovine serum albumin (BSA-ff; CultureSure albumin, Wako Pure Chemical Industries, Ltd.) at a final concentration of 5 mg/mL and Y-27632 (Wako Pure Chemical Industries, Ltd.) at a final concentration of 10 µM. In addition, the following lipids were each added to the cell suspension and the cells were subjected to rotary shaking culture for 1 day under the same conditions as in Example 2 to culture human iPS cells in suspension. After one day of the suspension culture, the cells were observed under a microscope.

The lipids and additives used in this study are as follows:
· LPA (Lysophosphatidic acid) (1-O-9Z-octadecenoyl-sn-glyceryl-3-phosphoric acid, a sodium salt, Cat. No. 62215, Cayman, Inc.; with an oleyl group at sn-1 position).
· S1P (Sphingosine-1-phosphoric acid) (2S-amino-1-(dihydrogen phosphate)-4E-octadecene-1,3R-diol, Cat. No. 62570, Cayman, Inc.)
LPA at 0.2 µg/mL or S1P at 0.2 µg/mL was added to the human iPS cell suspension.

A control test was conducted using the cell suspension prepared under the same conditions as above except that the above lipids were not added.

### (Results)

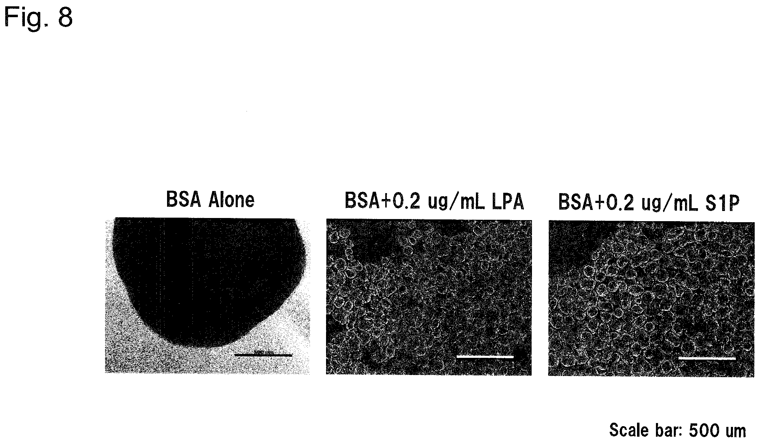

Figure 8 shows micrographs after the above suspension culture. The observation results demonstrated that in the control test, large aggregates (with a diameter of 1 mm or larger) were formed; and when the cell suspension contained 0.2 µg/mL of LPA (lysophosphatidic acid) or S1P (sphingosine-1-phosphoric acid), a large number of spherical cell aggregates with a substantially uniform size of about 100 µm were formed.

### <Example 6: Concentration of LPA or S1P Added and Size of Aggregates>

In Example 5, LPA and S1P were found to exert an ability to inhibit cell aggregation. Here, cell suspensions containing different concentrations of LPA or S1P added were used to perform suspension culture.

### (LPA Concentration)

Human iPS cell suspensions were prepared using substantially the same protocol as in Example 5. LPA was added as a sodium salt at a concentration of 0.00128 µg/mL, 0.0064 µg/mL, 0.032 µg/mL, 0.16 µg/mL, 0.8 µg/mL, 4 µg/mL, 20 µg/mL, or 100 µg/mL. To each suspension were added BSA-ff at a final concentration of 5 mg/mL and Y-27632 (Wako Pure Chemical Industries, Ltd.) at a final concentration of 10 µM. The cells were cultured in suspension for 1 day under the same conditions as in Example 5 and then observed under a microscope.

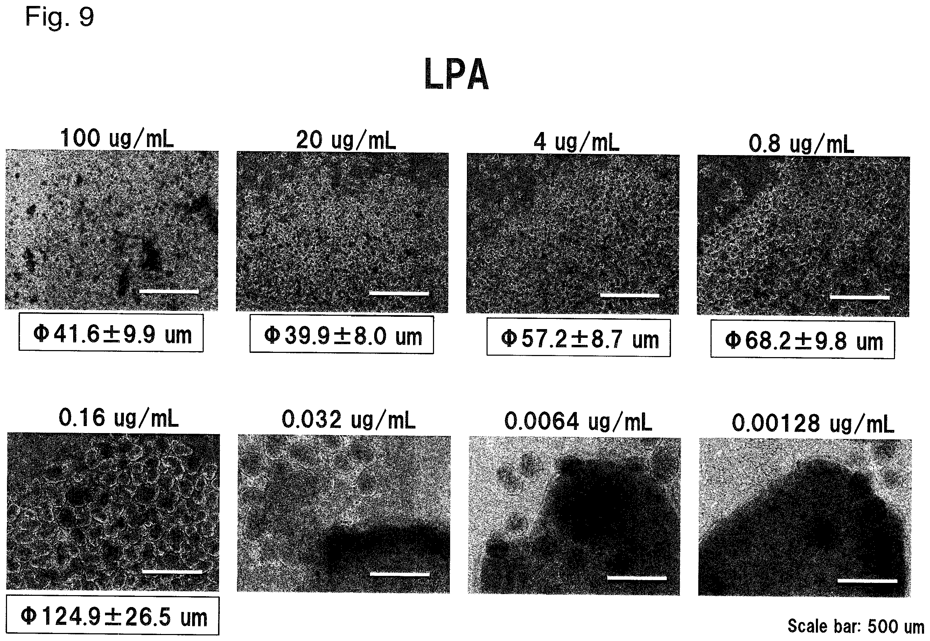

Figure 9 shows the observation results. The size of each cell aggregate changed depending on the concentration of LPA. When the concentration of LPA was from 0.16 to 100 µg/mL, cell aggregates with a substantially uniform size were formed. When the concentration of LPA is 0.032 µg/mL or less, large cell aggregates with a diameter of 1 mm or larger were formed.

Inspected were 30 cell aggregates on an observation image obtained when the concentration of LPA was from 0.16 to 100 µg/mL. While being compared using a micrograph scale, the width (referred to as "φ") of the widest portion of each cell aggregate was measured to calculate an average ± standard deviation.

When the concentration of LPA was 0.16 µg/mL, φ = 124.9 ± 26.5 µm; at 0.8 µg/mL, φ = 68.2 ± 9.8 µm; at 4 µg/mL, φ = 57.2 ± 8.7 µm; at 20 µg/mL, φ = 39.9 ± 8.0 µm; and at 100 µg/mL, φ = 41.6 ± 9.9 µm.

### (S1P Concentration)

Human iPS cell suspensions were prepared using substantially the same protocol as in Example 5. S1P was added as a free form at a concentration of 0.00128 µg/mL, 0.0064 µg/mL, 0.032 µg/mL, 0.16 µg/mL, 0.8 µg/mL, 4 µg/mL, 20 µg/mL, or 100 µg/mL. To each suspension were added BSA-ff at a final concentration of 5 mg/mL and Y-27632 (Wako Pure Chemical Industries, Ltd.) at a final concentration of 10 µM. The cells were cultured in suspension for 1 day under the same conditions as in Example 5 and then observed under a microscope.

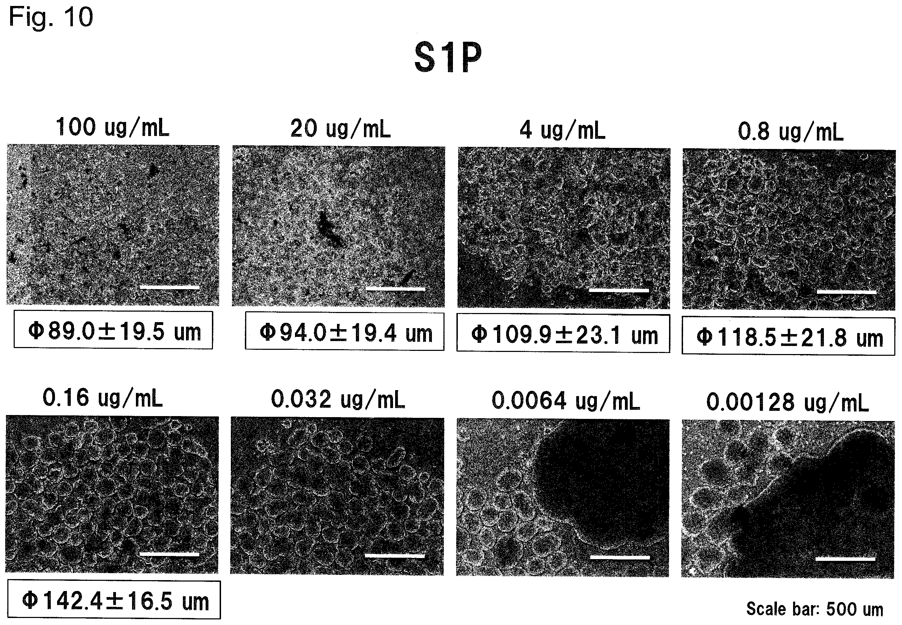

Figure 10 shows the observation results. The size of each cell aggregate changed depending on the concentration of S1P. When the concentration of S1P was from 0.032 to 100 µg/mL, cell aggregates with a substantially uniform size were formed. When the concentration of S1P is 0.0064 µg/mL or less, large cell aggregates with a diameter of 1 mm or larger were formed.

Inspected were 30 cell aggregates on an observation image obtained when the concentration of S1P was from 0.032 to 100 µg/mL. While being compared using a micrograph scale, the width (referred to as "φ") of the widest portion of each cell aggregate was measured to calculate an average ± standard deviation.

When the concentration of S1P was 0.16 µg/mL, φ = 142.4 ± 16.4 µm; at 0.8 µg/mL, φ = 118.5 ± 21.8 µm; at 4 µg/mL, φ = 109.9 ± 23.1 µm; at 20 µg/mL, φ = 94.0 ± 19.4 µm; and at 100 µg/mL, φ = 89.0 ± 19.5 µm.

### <Example 7: Effect of the Presence of LPA or S1P on Cell Proliferation Potential and Undifferentiated State>

Human iPS cells were cultured in suspension under culture conditions in the presence of LPA or S1P at different concentrations. Next, the glucose consumption, the total cell count, and the percentage of cells positive for undifferentiation markers were determined. How these additives affected the cells was analyzed.

### (Protocol)

Human iPS cell suspensions were prepared using substantially the same protocol as in Example 5. To each cell suspension were added LPA or S1P at a final concentration of 0.2 µg/mL or 1 µg/mL, and BSA-ff at a final concentration of 5 mg/mL and Y-27632 (Wako Pure Chemical Industries, Ltd.) at a final concentration of 10 µM. As a control, cell suspensions were prepared using the same protocol as in Example 5. That is, prepared were the cell suspensions solely containing BSA-ff at a final concentration of 5 mg/mL and Y-27632 at a final concentration of 10 µM.

The above cell suspensions were subjected to suspension culture for 2 days under the same conditions as in Example 5. At Day 2 of culture, the culture medium was changed every day with Essential 8^{™} culture medium supplemented with 5 mg/mL BSA-ff. At Days 2, 3, 4, and 5, the concentration of glucose in the culture supernatant was measured to calculate glucose consumption. In addition, at Day 5 of culture, cell aggregates were collected, dispersed using Accutase, and suspended in Essential 8^{™} culture medium supplemented with 5 mg/mL BSA-ff. A portion of each cell suspension was stained with trypan blue and the number of cells was counted. The above cell suspensions were centrifuged at 300 g for 5 min, the supernatant was then removed, and the cells were washed with PBS (phosphate buffered saline). Next, the cells were fixed with 4% PFA (paraformaldehyde) at room temperature for 20 min, then washed 3 times with PBS, and permeabilized with cold methanol at -20°C overnight. After washed 3 times with PBS, the cells were blocked with 3% FBS (fetal calf serum)/PBS and stained using a fluorescently labeled anti-SOX2 antibody (Cat. No. 656110, Biolegend, Inc.) and a fluorescently labeled anti-OCT4 antibody (Cat. No. 653703, Biolegend, Inc.) at 4°C for 1 h. After washed once with 3% FBS (fetal calf serum)/PBS, the cells were made to pass through a cell strainer. The resulting cells were analyzed on FACSVerse.

The following procedure was used to measure glucose consumption. Specifically, the culture supernatant was recovered at medium change and a bioanalyzer (YSI2950), manufactured by YSI, Inc., was used to measure the remaining glucose amount. In this way, the glucose consumption was calculated.

A total cell count was measured at Day 5 of culture. The following procedure was used to measure the total cell count. Specifically, the cell aggregates that had been formed were treated with TrypLE Select for 5 to 10 min, pipetted using a blue tip to monodisperse as cells, and stained with trypan blue. After that, the number of cells was counted using a hemocytometer to determine the total cell count.

### (Results)

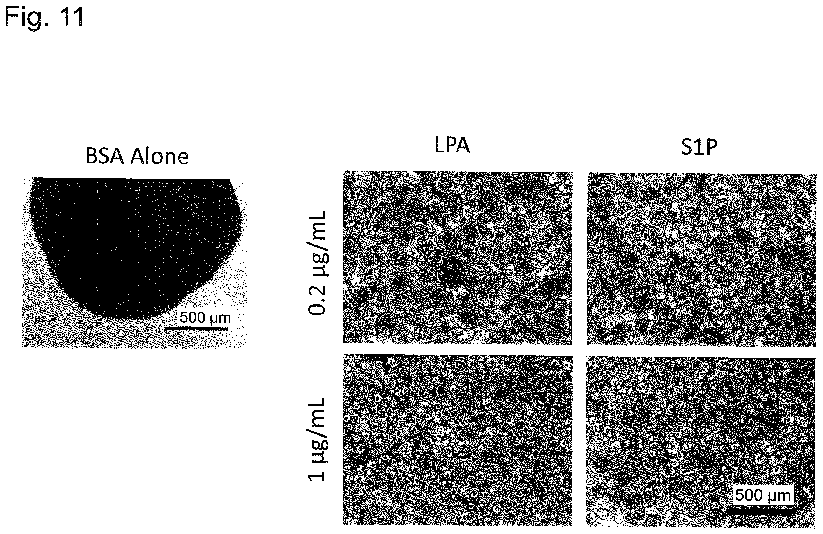

The pictures of Figure 11 are micrographs obtained at Day 2 after the start of culture. When LPA and S1P were each added and tested, cell aggregates with an appropriate size (with a diameter of 500 µm or less) were formed. By contrast, when BSA-ff alone was added and tested, large cell aggregates (with a diameter of 1 mm or more) were formed.

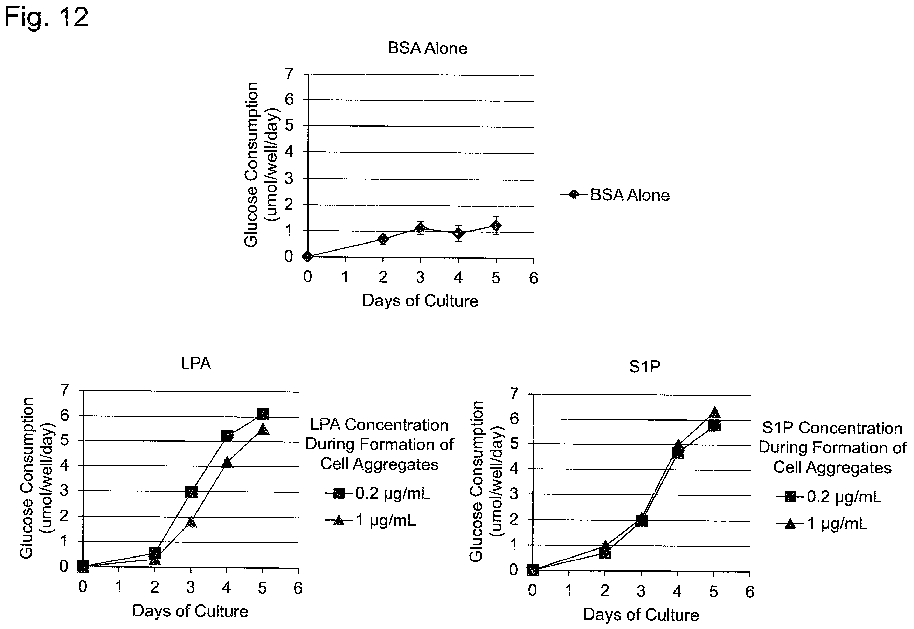

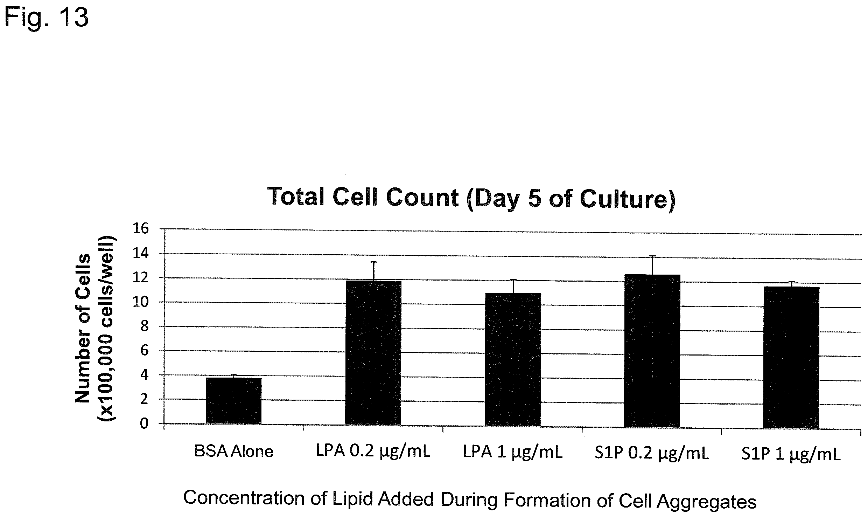

Figure 12 shows the results of measuring glucose consumption. Figure 13 shows the total cell count at Day 5 of culture. The glucose consumption and the number of cells were larger in the case of the addition of LPA or S1P than the case of the addition of BSA-ff alone. This revealed that the cells proliferated remarkably when LPA or S1P was added.

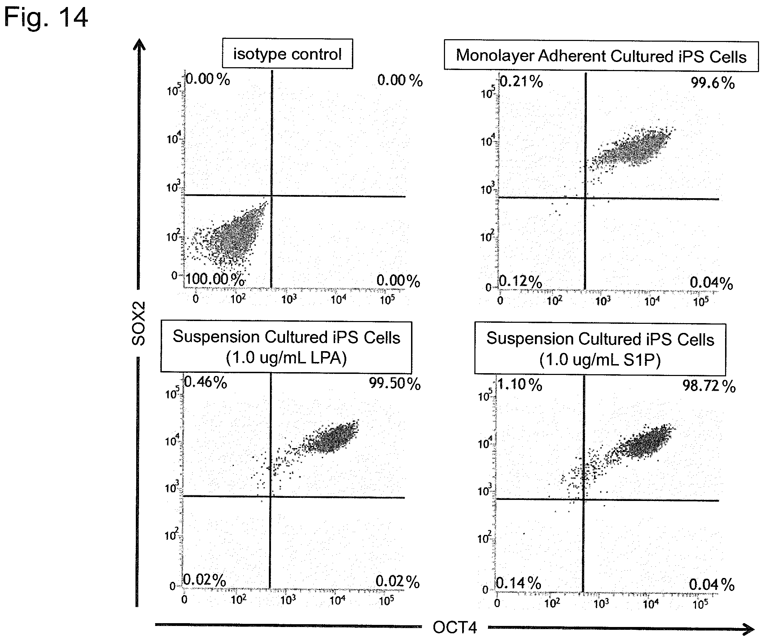

Figure 14 shows the results of measuring the percentage of cells positive for undifferentiation markers. When the cells were cultured as a suspension containing 1 µg/mL of LPA or S1P, 95% or more of the cells expressed undifferentiation markers OCT4 and SOX2, which is similar to cells in monolayer adherent culture. This verified that the human iPS cell aggregates that had been formed by the addition of the lipid remained undifferentiated.

### <Example 8: Examination of How Culture Can be Split and Scaled Up>

Human iPS cell suspensions were prepared using substantially the same protocol as in Example 5. AlbuMAX^{™} II was added at a final concentration of 5 mg/mL, BSA-ff was added at a final concentration of 5 mg/mL, and Y-27632 was added at a final concentration of 10 µM. The volume of culture medium was set to 4 mL per well, and the cells were seeded on a 6-well plate (Sumitomo Bakelite Co., Ltd.) at a cell density of 2 × 10⁵ cells per ml. The plate was rotated on a rotary shaker (OPTIMA, Inc.) at a speed of 90 rpm and cells were cultured under conditions at 5% CO₂ and 37°C for 2 days to form aggregates. Then, the culture medium was replaced every day for 4 days by Essential 8^{™} culture medium containing a final concentration of 0.5 mg/mL AlbuMAX^{™} II and a final concentration of 5 mg/mL BSA-ff. The following procedure was used to split cells at 6 days after the cell seeding. Cell aggregates were collected and washed once with PBS. Next, the cell aggregates were subjected to Accutase treatment at 37°C for 10 min to disperse the cells. Then, Essential 8^{™} culture medium containing a final concentration of 5 mg/mL BSA-ff was added. After the mixture was centrifuged at 300 g for 3 min and the supernatant was removed, human iPS cell suspensions (supplemented with a final concentration of 5 mg/mL AlbuMAX^{™} II, a final concentration of 5 mg/mL BSA-ff, and a final concentration of 10 µM Y-27632) were likewise prepared. The cells were seeded in a 1-L Erlenmeyer flask (Corning, Inc., product No. 431147) such that the volume of culture medium was 300 mL per flask and the cell density was 2 × 10⁵ cells per ml. The cells were cultured like the case of the above 6-well plate. At 5 days after the seeding, human iPS cell suspensions (supplemented with a final concentration of 5 mg/mL AlbuMAX^{™} II, a final concentration of 5 mg/mL BSA-ff, and a final concentration of 10 µM Y-27632) were likewise prepared. The cells were seeded and split in a 3-L Erlenmeyer flask (Corning, Inc., product No. 431252) such that the volume of culture medium was 1.6 L per flask and the cell density was 2 × 10⁵ cells per ml. The speed of rotation was set to 70 rpm and substantially the same procedure as above was used to culture the cells. For each culture volume (at 4-mL scale, 300-mL scale, and 1.6-L scale), micrographs of cell aggregates at 1, 5, or 6 days after the seeding were taken. In addition, the same procedure as in Example 2 was used to count the number of cells at the final day of culture. Also, the same procedure as in Example 7 was used to analyze the percentage of cells positive for the undifferentiation markers. As a control, adherent cultured cells as obtained using the procedure of Example 1 were used.

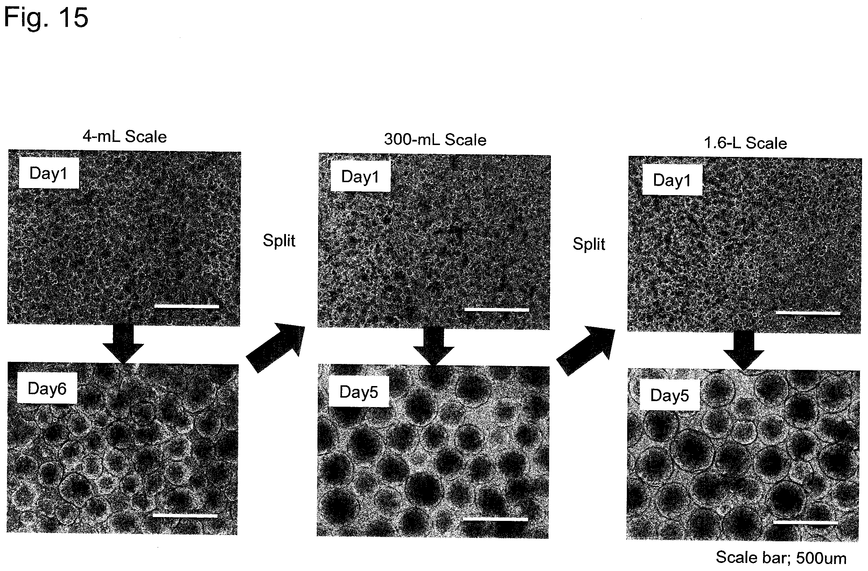

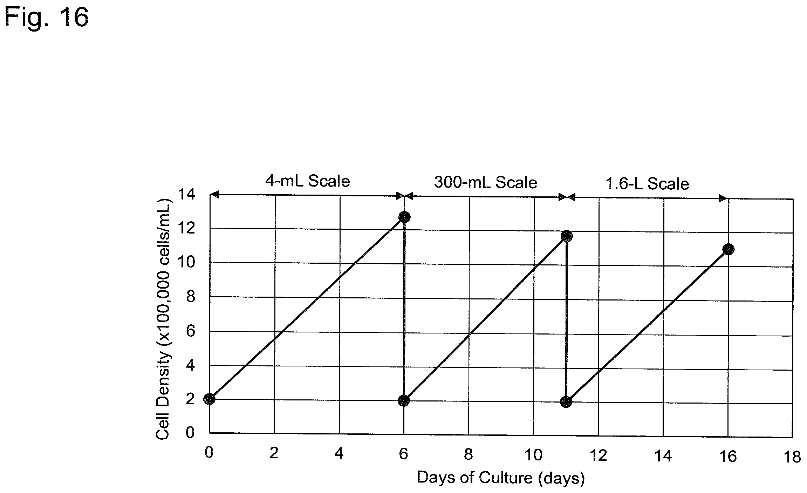

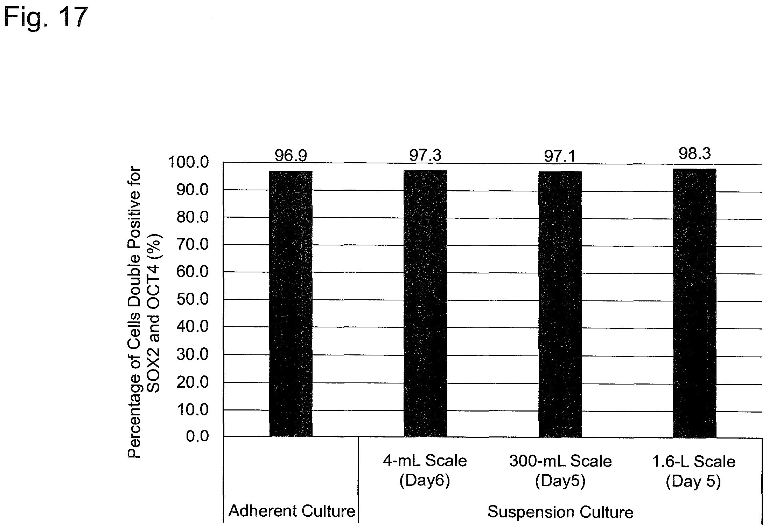

The above microscopic observation results have demonstrated that regardless of the culture scale, cell aggregates with a substantially uniform size can be likewise formed and that the cells can proliferate while remaining undifferentiated (Figures 15, 16, and 17).

## Claims

1. A method for producing cell aggregates, comprising a step of culturing cells while suspended in a liquid culture medium comprising a lysophospholipid,
wherein the cells are pluripotent stem cells, wherein the liquid culture medium comprises the lysophospholipid in an amount of more than 0.0064 µg/mL and 100 µg/mL or less,
wherein the lysophospholipid is at least one of lysophosphatidic acid and sphingosine-1-phosphoric acid.

2. The method according to claim 1, further comprising at least one of a step of adding the lysophospholipid to prepare the liquid culture medium and a step of generating the lysophospholipid through an enzymatic reaction to prepare the liquid culture medium.

3. The method according to any one of claims 1 or 2, wherein the cells are cells isolated after undergoing adherent or suspension culture.

4. The method according to any one of claims 1 to 3, wherein the liquid culture medium comprises at least one selected from the group consisting of L-ascorbic acid, insulin, transferrin, selenium, and sodium bicarbonate.

5. The method according to any one of claims 1 to 4, wherein the liquid culture medium comprises a growth factor.

6. The method according to claim 5, wherein the growth factor is at least one of FGF2 and TGF-β1.

7. The method according to any one of claims 1 to 6, wherein the liquid culture medium comprises a ROCK inhibitor.

8. In vitro use of a lysophospholipid for producing cell aggregates
wherein the cells are pluripotent stem cells, wherein the lysophospholipid is present in a concentration of more than 0.0064 µg/mL and 100 µg/mL or less,
wherein the lysophospholipid is at least one of lysophosphatidic acid and sphingosine-1-phosphoric acid.

9. The use according to claim 8, wherein the lysophospholipid is used in combination with a growth factor.

10. The use according to claims 9, wherein the growth factor is at least one of FGF2 and TGF-β1.

11. The use according to any one of claims 8 to 10, wherein the lysophospholipid is used in combination with a ROCK inhibitor.

## Patentansprüche

1. Verfahren zum Herstellen von Zellaggregaten, umfassend einen Schritt des Kultivierens von Zellen, während diese in einem flüssigen Kulturmedium suspendiert sind, das ein Lysophospholipid umfasst,
wobei die Zellen pluripotente Stammzellen sind, wobei das flüssige Kulturmedium das Lysophospholipid in einer Menge von mehr als 0,0064 µg/ml und 100 µg/ml oder weniger umfasst,
wobei das Lysophospholipid mindestens eines von Lysophosphatidsäure und Sphingosin-1-phosphorsäure ist.

2. Verfahren gemäß Anspruch 1, ferner umfassend mindestens einen Schritt des Hinzufügens des Lysophospholipids zum Herstellen des flüssigen Kulturmediums oder einen Schritt des Erzeugens des Lysophospholipids durch eine enzymatische Reaktion zum Herstellen des flüssigen Kulturmediums.

3. Verfahren gemäß irgendeinem der Ansprüche 1 oder 2, wobei die Zellen Zellen sind, die nach Durchlaufen einer Adhäsions- oder Suspensionskultur isoliert wurden.

4. Verfahren gemäß irgendeinem der Ansprüche 1 bis 3, wobei das flüssige Kulturmedium mindestens eines ausgewählt aus der Gruppe bestehend aus L-Ascorbinsäure, Insulin, Transferrin, Selen und Natriumbicarbonat umfasst.

5. Verfahren gemäß irgendeinem der Ansprüche 1 bis 4, wobei das flüssige Kulturmedium einen Wachstumsfaktor umfasst.

6. Verfahren gemäß Anspruch 5, wobei der Wachstumsfaktor mindestens einer von FGF2 und TGF-β1 ist.

7. Verfahren gemäß irgendeinem der Ansprüche 1 bis 6, wobei das flüssige Kulturmedium einen ROCK-Inhibitor umfasst.

8. In-vitro-Verwendung eines Lysophospholipids zum Herstellen von Zellaggregaten,
wobei die Zellen pluripotente Stammzellen sind, wobei das Lysophospholipid in einer Konzentration von mehr als 0,0064 µg/ml und 100 µg/ml oder weniger vorliegt,
wobei das Lysophospholipid mindestens eines von Lysophosphatidsäure und Sphingosin-1-phosphorsäure ist.

9. Verwendung gemäß Anspruch 8, wobei das Lysophospholipid in Kombination mit einem Wachstumsfaktor verwendet wird.

10. Verwendung gemäß Ansprüche 9, wobei der Wachstumsfaktor mindestens einer von FGF2 und TGF-β1 ist.

11. Verwendung gemäß irgendeinem der Ansprüche 8 bis 10, wobei das Lysophospholipid in Kombination mit einem ROCK-Inhibitor verwendet wird.

## Revendications

1. Procédé de production d'agrégats cellulaires, comprenant une étape consistant à cultiver des cellules tout en les maintenant en suspension dans un milieu de culture liquide comprenant un lysophospholipide,
dans lequel les cellules sont des cellules souches pluripotentes, dans lequel le milieu de culture liquide comprend le lysophospholipide dans une quantité de plus de 0,0064 µg/mL et de 100 µg/mL ou moins,
dans lequel le lysophospholipide est au moins l'un de l'acide lysophosphatidique et de l'acide sphingosine-1-phosphorique.

2. Procédé selon la revendication 1, comprenant en outre au moins l'une d'une étape consistant à ajouter le lysophospholipide pour préparer le milieu de culture liquide et d'une étape de génération du lysophospholipide par une réaction enzymatique pour préparer le milieu de culture liquide.

3. Procédé selon l'une quelconque des revendications 1 ou 2, dans lequel les cellules sont des cellules isolées après avoir subi une culture adhérente ou une culture en suspension.

4. Procédé selon l'une quelconque des revendications 1 à 3, dans lequel le milieu de culture liquide comprend au moins un ou une choisi(e) dans le groupe constitué de l'acide L-ascorbique, l'insuline, la transferrine, le sélénium et le bicarbonate de sodium.

5. Procédé selon l'une quelconque des revendications 1 à 4, dans lequel le milieu de culture liquide comprend un facteur de croissance.

6. Procédé selon la revendication 5, dans lequel le facteur de croissance est au moins l'un de FGF2 et de TGF-β1.

7. Procédé selon l'une quelconque des revendications 1 à 6, dans lequel le milieu de culture liquide comprend un inhibiteur de ROCK.

8. Utilisation in vitro d'un lysophospholipide pour produire des agrégats cellulaires dans laquelle les cellules sont des cellules souches pluripotentes, dans laquelle le lysophospholipide est présent dans une concentration de plus de 0,0064 µg/mL et de 100 µg/mL ou moins,
dans laquelle le lysophospholipide est au moins l'un de l'acide lysophosphatidique et de l'acide sphingosine-1-phosphorique.

9. Utilisation selon la revendication 8, dans laquelle le lysophospholipide est utilisé en combinaison avec un facteur de croissance.

10. Utilisation selon la revendication 9, dans laquelle le facteur de croissance est au moins l'un de FGF2 et de TGF-β1.

11. Utilisation selon l'une quelconque des revendications 8 à 10, dans laquelle le lysophospholipide est utilisé en combinaison avec un inhibiteur de ROCK.
